# EUROPEAN PATENT SPECIFICATION

(11) **EP 4 057 004 B1**
(45) Date of publication and mention of the grant of the patent: **10.04.2024**
(21) Application number: 22161490.2
(22) Date of filing: 11.03.2022
(51) Int. Cl.: G01N 33/569, G01N 33/68

(54) **METHOD FOR ACQUIRING INFORMATION ON SPINAL MUSCULAR ATROPHY**
VERFAHREN ZUR ERFASSUNG VON INFORMATIONEN ÜBER SPINALE MUSKELATROPHIE
PROCÉDÉ D'ACQUISITION D'INFORMATIONS SUR L'AMYOTROPHIE SPINALE

(30) Priority: 12.03.2021 JP 2021040682
(43) Date of publication of application: 14.09.2022
(73) Proprietor: Tokyo Women's Medical University, Tokyo 162-8666 (JP); SYSMEX CORPORATION, Kobe-shi Hyogo 651-0073 (JP)
(72) Inventor: SAITO, Kayoko, Tokyo, 1628666 (JP); OTSUKI, Noriko, Tokyo, 1628666 (JP); MAEKAWA, Takanori, Kobe-shi, Hyogo, 651-0073 (JP)
(74) Representative: De Clercq & Partners

(56) References cited:
- EP-A1- 3 128 327
- PHILIP J. YOUNG ET AL: "The Relationship between SMN, the Spinal Muscular Atrophy Protein, and Nuclear Coiled Bodies in Differentiated Tissues and Cultured Cells", EXPERIMENTAL CELL RESEARCH, vol. 256, no. 2, 1 May 2000 (2000-05-01), pages 365-374, XP055389288, AMSTERDAM, NL ISSN: 0014-4827, DOI: 10.1006/excr.2000.4858
- HEBERT MICHAEL D. ET AL: "Coilin forms the bridge between Cajal bodies and SMN, the Spinal Muscular Atrophy protein", GENES & DEVELOPMENT, vol. 15, no. 20, 15 October 2001 (2001-10-15), pages 2720-2729, XP055946500, US ISSN: 0890-9369, DOI: 10.1101/gad.908401
- NORIKO OTSUKI ET AL: "A new biomarker candidate for spinal muscular atrophy: Identification of a peripheral blood cell population capable of monitoring the level of survival motor neuron protein", PLOS ONE, vol. 13, no. 8, 13 August 2018 (2018-08-13), page e0201764, XP055623513, DOI: 10.1371/journal.pone.0201764

## Description

### FIELD OF THE INVENTION

The present invention relates to a method for acquiring information on spinal muscular atrophy.

### BACKGROUND

Spinal muscular atrophy (SMA) is a neuromuscular disease caused by degeneration and loss of the motor neuron in the spinal cord, with symptoms of muscular atrophy and progressive muscle weakness. The causative gene for SMA is the survival motor neuron 1 (SMN1) gene. The SMN protein produced from the SMN1 gene plays an important role in formation and function maintenance of the motor neuron, etc. However, in SMA, an abnormality in the SMN1 gene significantly reduces the expression level of the SMN protein, resulting in degeneration and disappearance of the motor neuron.

U.S. Patent Application Publication No. 2017/0115297 discloses that the SMN protein is detected as a biomarker for diagnosing SMA and confirming the therapeutic effect. Specifically, U.S. Patent Application Publication No. 2017/0115297 discloses that a measurement is performed of the expression level of the SMN protein based on fluorescence intensity by labeling the SMN protein in a nucleated cell in a blood specimen with a fluorescent dye and detecting the dye with a flow cytometer.

U.S. Patent Application Publication No. 2017/0115297 uses the expression level of the SMN protein in a nucleated cell as an indicator. On the other hand, the present inventors have focused on the localization of the SMN protein in a nucleated cell. Therefore, it is an object of the present invention to provide a novel means for acquiring information on SMA based on an intracellular distance between the SMN protein and a predetermined nuclear protein localized in the nucleus of a nucleated cell. That is, an object of the present invention is to provide a method for acquiring information on spinal muscular atrophy.

### SUMMARY OF THE INVENTION

The present invention provides a method for acquiring information on spinal muscular atrophy, comprising: acquiring a fluorescence image of a monocyte in a measurement sample, wherein the measurement sample is a sample prepared from a blood specimen obtained from a subject, an SMN protein in the monocyte is labeled with a first fluorescent dye, and coilin in the monocyte is labeled with a second fluorescent dye, acquiring an intracellular distance between a first bright spot corresponding to the first fluorescent dye and a second bright spot corresponding to the second fluorescent dye in the fluorescence image, and acquiring a value regarding a number of monocytes in which the intracellular distance is equal to or less than a first threshold value, wherein the value is an indicator of spinal muscular atrophy affection, wherein:
when the value is less than a second threshold value, it is suggested that the subject suffers from spinal muscular atrophy, or
when the value is equal to or higher than a second threshold value, it is suggested that the subject does not suffer from spinal muscular atrophy.

The present invention provides a method for acquiring information on spinal muscular atrophy, comprising: acquiring a fluorescence image of a monocyte in a measurement sample, wherein the measurement sample is a sample prepared from a blood specimen obtained from a patient who has undergone a treatment for spinal muscular atrophy, an SMN protein in the monocyte is labeled with a first fluorescent dye, and coilin in the monocyte is labeled with a second fluorescent dye, acquiring an intracellular distance between a first bright spot corresponding to the first fluorescent dye and a second bright spot corresponding to the second fluorescent dye in the fluorescence image, and acquiring a value regarding a number of monocytes in which the intracellular distance is equal to or less than a first threshold value, wherein the value is an indicator of a response of the treatment for spinal muscular atrophy, wherein:
when the value is less than a second threshold value, it is suggested that the treatment for spinal muscular atrophy is not successful in the patient, or
when the value is equal to or higher than a second threshold value, it is suggested that the treatment for spinal muscular atrophy is successful in the patient.

The present invention provides a method for acquiring information on spinal muscular atrophy, comprising: acquiring a first fluorescence image of a monocyte in a first measurement sample and a second fluorescence image of a monocyte in a second measurement sample, wherein the first measurement sample is prepared from a blood specimen obtained from a patient before receiving a treatment for spinal muscular atrophy and a second measurement sample is prepared from a blood specimen obtained from the patient after receiving the treatment, an SMN protein in the monocyte in the first and second measurement samples is labeled with a first fluorescent dye, and coilin in the monocyte in the first and second measurement samples is labeled with a second fluorescent dye, acquiring a first intracellular distance between a first bright spot corresponding to the first fluorescent dye and a second bright spot corresponding to the second fluorescent dye in the first fluorescence image, and acquiring second intracellular distance between a first bright spot corresponding to the first fluorescent dye and a second bright spot corresponding to the second fluorescent dye in the second fluorescence image, and acquiring a first value regarding a number of monocytes in which the first intracellular distance is equal to or less than a first threshold value, and a second value regarding a number of monocytes in which the second intracellular distance is equal to or less than a second threshold value, wherein the first and second values are indicators of a response of the treatment for spinal muscular atrophy, wherein:
when the second value is less than the first value, it is suggested that the treatment for spinal muscular atrophy is not successful in the patient, or
when the second value is equal to or greater than the first value, it is suggested that the treatment for spinal muscular atrophy is successful in the patient.

In one embodiment of any of preceding methods, the intracellular distance is a distance between a center of gravity of the first bright spot and a center of gravity of the second bright spot in the fluorescence image.

In one embodiment of any of preceding methods, in the acquiring of an intracellular distance, when a plurality of the first bright spots and/or the second bright spots are present in one monocyte, a distance between the closest first bright spot and second bright spot is acquired as the intracellular distance.

In one embodiment of any of preceding methods, in the acquiring of an intracellular distance, when a plurality of the first bright spots and/or the second bright spots are present in one monocyte, distances between the first bright spot and second bright spot for all combinations of the first bright spot and second bright spot are acquired, and the distances are compared to acquire a shortest distance among the distances as the intracellular distance.

In one embodiment of any of preceding methods, the methods further comprise acquiring a number of monocytes comprising at least one first bright spot and at least one second bright spot in the fluorescence image, wherein the value is a ratio or a value of the ratio of a number of monocytes in which the intracellular distance is equal to or less than a first threshold value to the acquired number of monocytes.

According to the present invention, information on SMA in a subject can be acquired.

### BRIEF DESCRIPTION OF THE DRAWINGS

Fig. 1A is views schematically showing fluorescence images capturing a nucleated cell in which a first bright spot or a second bright spot is present.
Fig. 1B is views schematically showing fluorescence images capturing a nucleated cell in which a first bright spot or a second bright spot is present.
Fig. 2A is a schematic view showing an example of a reagent kit.
Fig. 2B is a schematic view showing an example of a reagent kit.
Fig. 3 is a schematic view showing an example of an acquisition device.
Fig. 4 is a 2D scattergram created based on the intensity of a fluorescence signal from a third fluorescent dye and the side scattered light intensity.
Fig. 5 is a schematic view showing a processing procedure for measuring an intracellular distance between a first bright spot and a second bright spot in a fluorescence image.
Fig. 6 is a schematic view showing a distance D between the centers of gravity of the center of gravity coordinate C1 of a first bright spot and the center of gravity coordinate C2 of a second bright spot.
Fig. 7A is a flowchart showing a determination procedure by an acquisition device o.
Fig. 7B is a flowchart showing a determination procedure by an acquisition device.
Fig. 7C is a flowchart showing a determination procedure by an acquisition device.
Fig. 8 is a 2D scattergram with the fluorescence intensity of PerCP/Cy5.5 (trademark) on the X-axis and the side scattered light intensity on the Y-axis.
Fig. 9 is an example of a fluorescence image and a transmitted light image of each monocyte in a monocyte fraction.
Fig. 10A is a graph showing the ratio of a monocyte having one or more bright spots of an SMN protein in monocyte fractions of healthy subjects and SMA patients.
Fig. 10B is a graph showing the ratio of a monocyte having two or more bright spots of an SMN protein in monocyte fractions of healthy subjects and SMA patients.
Fig. 10C is a graph showing the ratio of a monocyte having three or more bright spots of an SMN protein in monocyte fractions of healthy subjects and SMA patients.
Fig. 10D is a graph showing the ratio of a monocyte having one or more positions where a bright spot of an SMN protein and a bright spot of coilin are close to each other in monocyte fractions of healthy subjects and SMA patients.
Fig. 11 is a graph showing the ratio of a monocyte having one or more positions where a bright spot of an SMN protein and a bright spot of coilin are close to each other in a fraction of a patient who has been administered a therapeutic agent for SMA.
Fig. 12 is a graph showing the ratio of a monocyte having one or more positions where a bright spot of an SMN protein and a bright spot of coilin are close to each other in a fraction of a patient who has been administered a therapeutic agent for SMA, and the serum CK activity of the patient.

### DETAILED DESCRIPTION OF THE PREFERRED EMBODIMENTS

According to the method for acquiring information on SMA of the present invention (hereinafter, also referred to as "acquisition method"), a value that is an indicator of SMA affection can be acquired. In the acquisition method of the present invention, first, a fluorescence image of a monocyte in a measurement sample is acquired.

The measurement sample is a sample prepared from a blood specimen obtained from a subject. In one embodiment, a fraction containing a monocyte is acquired from a blood specimen obtained from a subject, and the monocyte in the fraction is stained with a first fluorescent dye and a second fluorescent dye, with the result that a sample containing a monocyte in which the SMN protein is labeled with the first fluorescent dye, and coilin is labeled with the second fluorescent dye can be obtained as the measurement sample.

The subject is not particularly limited, and examples thereof include a person suspected of suffering from SMA and a person whose close relative is an SMA patient. Specific examples include a person who has decreased motor function such as difficulty in standing up and walking without support, or SMA symptom such as muscle atrophy or dyspnea, and a person who is suspected of having SMA by genetic testing or molecular marker testing. The subject may be a person who does not exhibit any abnormality in motor function.

The blood specimen may be whole blood, or a fraction prepared from whole blood and containing a monocyte. Examples of such a fraction include a buffy coat, a fraction containing a peripheral blood mononuclear cell (PBMC), and a fraction containing a nucleated cell obtained by hemolyzing and centrifuging an erythrocyte. Peripheral blood is preferably as whole blood. Whole blood may contain a publicly known anticoagulant such as heparin, EDTA salt or sodium citrate. When whole blood is collected from a subject as the blood specimen, the amount is about 0.5 mL or more, preferably about 1 mL or more, and about 3 mL or less, preferably 2 mL or less.

Nucleated cell includes a leukocyte cell, a hematopoietic stem cell and a vascular endothelial progenitor cell. Examples of a leukocyte cell include a monocyte, a lymphocyte, a neutrophil, an eosinophil and a basophil. The lymphocyte includes a T cell, a B cell and a natural killer (NK) cell. In the methods of the invention, the nucleated cell is a monocyte.

When the blood specimen is whole blood, it is preferable to acquire a fraction containing a monocyte from whole blood for preparation of the measurement sample. For example, a fraction containing a nucleated cell can be acquired by adding a publicly known hemolytic agent to whole blood to hemolyze an erythrocyte and precipitating a nucleated cell by centrifugation. A buffy coat can be acquired as a fraction containing a nucleated cell by centrifuging whole blood as it is and fractionating the intermediate layer. A fraction containing a PBMC can be acquired as a fraction containing a nucleated cell by adding a separation medium such as Ficoll to whole blood and performing density gradient centrifugation.

Using the acquired fraction containing a nucleated cell, the measurement sample can be prepared by staining the monocyte with the first fluorescent dye and the second fluorescent dye. In a preferred embodiment, the acquisition method includes staining a monocyte with a first fluorescent dye and a second fluorescent dye prior to the acquiring of a fluorescence image. The staining labels the SMN protein in the monocyte with the first fluorescent dye, and coilin in the monocyte with the second fluorescent dye. In the methods of the present invention, the excitation wavelength of the first fluorescent dye is different from the excitation wavelength of the second fluorescent dye, and the fluorescence wavelength of the first fluorescent dye is different from the fluorescence wavelength of the second fluorescent dye.

The fluorescent dye is not particularly limited, but can be appropriately selected from publicly known fluorescent dyes. Examples thereof include fluorescein isothiocyanate (FITC), rhodamine, coumarin, an imidazole derivative, an indole derivative, allophycocyanin (APC), phycoerythrin (PE), PerCP/Cy5.5 (trademark), Alexa Fluor (registered trademark), Cy3 (registered trademark), Cy5 (registered trademark), Cy5.5 (registered trademark), Cy7 (registered trademark) and DyLight (registered trademark) Fluor.

The SMN protein is known to migrate into the nucleus of a cell and localize in the nucleolus. The nucleolus is not particularly limited, but examples thereof include the Cajal body. Coilin itself is publicly known, and its amino acid sequence can be acquired from a publicly known database such as NCBI (National Center for Biotechnology Information). Coilin is known as a protein localized in the Cajal body.

In the methods of the present invention, it is preferable to label the SMN protein in a monocyte with the first fluorescent dye via a substance capable of specifically binding to the SMN protein. In the methods of the present invention, it is preferable to label the coilin in a monocyte with the second fluorescent dye via a substance capable of specifically binding to coilin. Each of the substances capable of specifically binding to each of the proteins include an antibody and an aptamer. Among them, an antibody is particularly preferable.

As used herein, the term "antibody" includes a full-length antibody and a fragment thereof. Examples of the fragment of an antibody include a reduced IgG (rIgG), a Fab, a Fab', a F(ab')2, a Fv, a single-chain antibody (scFv), a diabody and a triabody. The antibody may be either a monoclonal antibody or a polyclonal antibody. For example, an antibody capable of specifically binding to the SMN protein and an antibody capable of specifically binding to coilin are publicly known. These antibodies are generally available. Alternatively, in order to acquire an antibody capable of specifically binding to each of the proteins, a hybridoma that produces the antibody may be prepared using the method described in Kohler G. and Milstein C., Nature, vol.256, pp.495-497, 1975. A commercially available antibody may be used.

The method for labeling a protein with a fluorescent dye using an antibody is referred to as an immunofluorescent staining method. The immunofluorescent staining method includes a direct method and an indirect method, and either of them may be used in the methods of the present invention. For example, the SMN protein may be labeled by the direct method and coilin may be labeled by the indirect method. Alternatively, the SMN protein may be labeled by the indirect method and coilin may be labeled by the direct method.

For example, when the SMN protein is labeled with the first fluorescent dye and coilin is labeled with the second fluorescent dye by the direct immunofluorescent staining method, a monocyte may be contacted with an anti-SMN protein antibody labeled with the first fluorescent dye and an anti-coilin antibody labeled with the second fluorescent dye. Specifically, a fraction containing the monocyte is mixed with a solution of each of the labeled antibodies, followed by incubation under a predetermined condition. The method itself for labeling an antibody with a fluorescent dye is publicly known. For example, the antibody and the fluorescent dye may be linked by a covalent bond using a cross-linking agent or the like. The temperature and time condition for contact is not particularly limited, but for example, the mixture may be incubated at 4°C to 42°C for 1 minute to 24 hours. Incubation may be performed in the dark to prevent fading of the fluorescent dye.

For example, when the SMN protein is labeled with the first fluorescent dye and coilin is labeled with the second fluorescent dye by the indirect immunofluorescent staining method, first, a monocyte is contacted with an anti-SMN protein antibody and an anti-coilin antibody. Specifically, a fraction containing the monocyte is mixed with a solution of each of the antibodies, followed by incubation under a predetermined condition. Since a secondary antibody is used in the indirect method, the anti-SMN protein antibody and the anti-coilin antibody are preferably antibodies derived from different animal species. Then, a monocyte that has contacted with the anti-SMN protein antibody and the anti-coilin antibody is contacted with a secondary antibody labeled with the first fluorescent dye against the anti-SMN protein antibody and a secondary antibody labeled with the second fluorescent dye against the anti-coilin antibody. Specifically, a solution containing the monocyte is mixed with a solution of each of the labeled secondary antibodies, followed by incubation under a predetermined condition. The incubation condition is as described above.

In a further embodiment, the anti-SMN protein antibody or anti-coilin antibody may be labeled with biotin. In this case, the biotin-modified anti-SMN protein antibody or biotin-modified anti-coilin antibody, and avidin on which the first or second fluorescent dye is immobilized are used in immunostaining. Through the specific binding of biotin to avidin, the first or second fluorescent dye can indirectly bind to the antibody bound to the SMN protein or coilin. The biotin includes biotin itself and a biotin analog such as desthiobiotin and oxybiotin. The avidin includes avidin itself and an avidin analog such as streptavidin and Tamavidin (registered trademark).

In the methods of the present invention, after contact between the monocyte and the respective antibodies, the cell may be washed to remove the unreacted antibodies. A suitable aqueous medium such as PBS can be used for washing. A commercially available washing buffer may be used.

In particular embodiments of the methods of the present invention, the membrane permeation treatment may be performed for the monocyte prior to staining, because the SMN protein and tcoilin in the cell are stained. By this treatment, the cell membrane and nuclear envelope are damaged to the extent that the fluorescent dyes and the substances capable of specifically binding to the respective proteins can pass through, so that the fluorescent dyes and the substances capable of specifically binding to the respective proteins can migrate into the nucleus of the monocyte. The membrane permeation treatment can be performed, for example, by contacting a monocyte with a treatment liquid that causes damage to the cell membrane and the nuclear membrane to the extent that the fluorescent dyes can pass through. Such a treatment liquid is preferably a solution of a surfactant. The surfactant commonly used in the membrane permeation treatment is publicly known, and examples thereof include Nonidet (registered trademark) P-40 and Triton (registered trademark) X-100. The solvent includes water, saline or a suitable buffer solution. Examples of the buffer solution include a phosphate buffer solution (PBS) and a Good's buffer solution. Examples of the Good's buffer solution include PIPES, MES, Bis-Tris, ADA, Bis-Tris-Propane, ACES, MOPS, MOPSO, BES, TES, HEPES, HEPPS, Tricine, Tris, Bicine and TAPS.

If necessary, the nuclei of a monocyte may be labeled. When a monocyte has been subjected to the membrane permeation treatment, the nuclei of the cell can be stained with a fluorescent dye capable of staining a nucleic acid (hereinafter, also referred to as "nuclear staining dye"). Examples of such a fluorescent dye include Hoechst33342, Hoechst33258 and 4',6-diamidino-2-phenylindole dihydrochloride (DAPI).

If necessary, a monocyte may be immobilized prior to the membrane permeation treatment of the monocyte. The method itself for immobilizing a cell is publicly known. The method can be performed, for example, by contacting a monocyte with an immobilizing solution. Such an immobilizing solution includes paraformaldehyde, formaldehyde, glutaraldehyde, acetone, methanol, ethanol, and a combination thereof. A commercially available cell-immobilizing solution may be used. When whole blood is used as the specimen, an immobilizing solution containing a hemolytic agent may be used.

In particular embodiments of the methods of the present invention, a blocking treatment may be performed on the Fc receptor on the surface of a monocyte before the immobilizing or membrane permeation treatment on the monocyte. The blocking treatment is a treatment for suppressing a non-specific reaction of an antibody to a cell via an Fc receptor during immunofluorescent staining. The blocking treatment can be performed by contacting a monocyte with a reagent for the blocking treatment. The reagent itself is publicly known, and examples thereof include an anti-Fc receptor antibody and serum. A commercially available reagent may be used.

As mentioned above, a nucleated cell in blood includes various types of cells. When applying the acquisition method of the present invention to a mononcyte, the monocyte may be selected using a surface antigen marker for the monocyte. Specifically, the surface antigen marker for a monocyte is labeled with a third fluorescent dye, and the monocyte is selected based on the fluorescent signal from the third fluorescent dye. Labeling of the surface antigen marker with the third fluorescent dye can be performed using a substance capable of specifically binding to the surface antigen marker (for example, an antibody and an aptamer). As described above, the acquisition method of the present invention may include staining a monocyte with a third fluorescent dye prior to the acquiring of a fluorescence image. The acquisition method of the present invention may include selecting a monocyte based on the fluorescent signal from cells labeled with the third fluorescent dye.

If necessary, the fluorescent signal from the third fluorescent dye may be combined with the side scattered light intensity. For example, a 2D scattergram may be created having the fluorescence intensity of the third fluorescent dye on the X-axis and the side scattered light intensity on the Y-axis, and monocytes may be selected on this 2D scattergram. Accordingly, the acquisition method of the present invention may include selecting a monocyte based on the fluorescent signal and the side scattered light signal from cells labeled with the third fluorescent dye.

The surface antigen marker itself for a monocyte is publicly known. Generally, CD14 is known as a monocyte marker, but they are not limited thereto.

In the methods of the present invention, it is preferable to acquire a fluorescence image of a monocyte in a measurement sample by a means for observing and recording the fluorescence generated by irradiating cells stained with a fluorescent dye with excitation light as an image. Such a means is not particularly limited, but a means for observing the localization of a fluorescently labeled protein in each cell is preferable. In one embodiment, a fluorescence image of a monocyte in a measurement sample is acquired with a fluorescence microscope or a flow cytometer. The flow cytometer includes an imaging flow cytometer (IFC).

In the methods of the present invention, a plurality of monocytes may be captured in one fluorescence image. For example, using a fluorescence microscope, it is possible to acquire one fluorescence image in which a plurality of monocytes is captured. A plurality of fluorescence images in which a plurality of monocytes is captured may be acquired. The magnification of the fluorescence image is not particularly limited, but it is preferable that the magnification be such that the localization of an SMN protein and coilin that have been fluorescently labeled can be observed in each monocyte.

In a further embodiment, a plurality of fluorescence images may be acquired, and one monocyte may be captured in the respective fluorescence images between which the cells are different from each other. For example, using an IFC, it is possible to acquire fluorescence images of individual monocytes. The IFC is a flow cytometer equipped with an imaging part. The IFC is a device capable of acquiring an image of a cell flowing in a liquid. More specifically, the IFC can acquire and quantitatively measure a fluorescence signal, a scattered light signal, a fluorescence image and a transmitted light image from each of thousands to millions of cells in a short time of seconds to minutes. Information on each cell can be extracted by image processing.

The fluorescence microscope and the flow cytometer are not particularly limited, but a commercially available device may be used. The light source is not particularly limited, but a light source having a wavelength suitable for exciting the fluorescent dye can be appropriately selected. As the light source, for example, a blue semiconductor laser, a red semiconductor laser, an argon laser, an He-Ne laser or a mercury arc lamp is used.

In the acquisition method of the present invention, the intracellular distance between a first bright spot corresponding to the first fluorescent dye and a second bright spot corresponding to the second fluorescent dye is acquired (or determined) in the fluorescence image. Generally, a fluorescence image is composed of a pixel having a pixel value corresponding to the signal intensity of fluorescence emitted by the fluorescent dye (fluorescence intensity). As used herein, the "bright spot" refers to a point of fluorescence emitted by a fluorescent dye labeling a protein, in which the pixel value of each pixel constituting the point is equal to or higher than a predetermined value. The first bright spot corresponding to the first fluorescent dye (hereinafter referred to as "first bright spot") represents one or more molecules of the SMN protein labeled with the first fluorescent dye. The second bright spot corresponding to the second fluorescent dye (hereinafter referred to as "second bright spot") represents one or more molecules of coilin labeled with the second fluorescent dye.

In the fluorescence image, the number of monocytes containing at least one first bright spot and at least one second bright spot may be acquired (or determined). A monocyte in which both the first bright spot and the second bright spot are present may be visually selected or selected by a device that has acquired the fluorescence image. In a preferred embodiment, the number of monocytes containing at least one first bright spot and at least one second bright spot is acquired (or determined).

In the fluorescence image, the intracellular distance between the first bright spot and the second bright spot in at least one monocyte is acquired (or determined). The number of monocytes for which the intracellular distance is examined is not particularly limited, but the larger the number, the more accurate information can be acquired. In a preferred embodiment, in the fluorescence image, the intracellular distance between the first bright spot and the second bright spot in each of the plurality of monocytes is acquired or determined.

In the methods of the present invention, the "intracellular distance between the first bright spot and the second bright spot" is a distance between the first bright spot and the second bright spot in one monocyte. The intracellular distance is a distance on the plane in the fluorescence image, in which the depth is not considered. The intracellular distance may be a physical distance or a parameter that reflects the physical distance. The physical distance is, for example, a distance expressed in metric units (for example, µm or nm). Examples of the physical distance include a value actually measured using a microscale, an eyepiece micrometer or the like, and a value measured by a device that has acquired the fluorescence image. Examples of the parameter that reflects the physical distance include the number of pixels between the first bright spot and the second bright spot in a fluorescence image captured at a predetermined magnification. The intracellular distance may be a physical distance converted from the parameter that reflects the physical distance.

Examples of the intracellular distance include a distance between positions where the outer circumferences of the first bright spot and the second bright spot are closest to each other, a distance between the centers of gravity of the first bright spot and the second bright spot, and a distance between the center points of the first bright spot and the second bright spot. In the fluorescence image, the "center of gravity of a bright spot" refers to a pixel located at the coordinate of the geometric center of gravity in an image showing a bright spot (hereinafter, also referred to as a "center of gravity coordinate"). In the fluorescence image, the "center point of a bright spot" refers to a pixel having the highest pixel value among pixels constituting an image showing a bright spot.

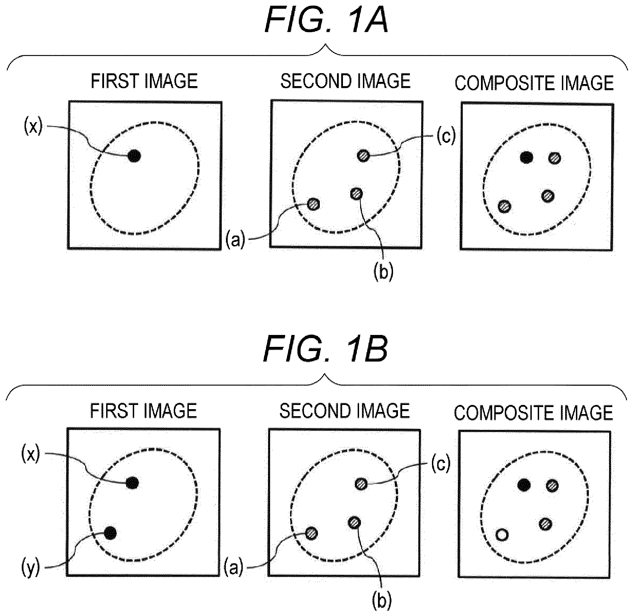

When there is a plurality of first bright spot and/or second bright spot in one monocyte, it is preferable to acquire (or determine, select) the closest distance between the first bright spot and the second bright spot as the intracellular distance. A description is made of acquisition of the intracellular distance with reference to Fig. 1. In the drawing, the broken line represents the outline of a nucleated cell. The first image and the second image are fluorescence images acquired based on the fluorescent signals emitted by the first and second fluorescent dyes, respectively. The composite image is an image in which the first image and the second image are superimposed. In Fig. 1A, the first image has a first bright spot (x), and the second image has second bright spots (a), (b) and (c). In the composite image, the distance between the first bright spot (x) and the second bright spot (a), the distance between the first bright spot (x) and the second bright spot (b), and the distance between the first bright spot (x) and the second bright spot (c) are calculated for comparison. The second bright spot closest to the first bright spot (x) is the second bright spot (c). In the example of Fig. 1A, the intracellular distance between the first bright spot (x) and the second bright spot (c) can be used.

In Fig. 1B, the first image has first bright spots (x) and (y), and the second image has second bright spots (a), (b) and (c). In the composite image, the bright spots represented by ∘ indicate a position where the first bright spot (y) overlaps with the second bright spot (a). This indicates that the SMN protein and coilin are co-localized. In the fluorescence image, the distances between the first bright spot and the second bright spot for all combinations of the first bright spot and the second bright spot are measured. More specifically, the distance between the first bright spot (x) and the second bright spot (a), the distance between the first bright spot (x) and the second bright spot (b), the distance between the first bright spot (x) and the second bright spot (c), the distance between the first bright spot (y) and the second bright spot (a), the distance between the first bright spot (y) and the second bright spot (b), and the distance between the first bright spot (y) and the second bright spot (c) are measured. Next, these distances are compared to identify the closest combination of the first bright spot and the second bright spot. In the example of Fig. 1B, the first bright spot (y) and the second bright spot (a) are the closest combination of the first bright spot and the second bright spot. In this example, the distance between the first bright spot (y) and the second bright spot (a) can be used as the intracellular distance.

In another embodiment, when there is a plurality of first bright spot and/or second bright spot in one monocyte, it is possible to acquire or obtain an average value of the distances between the first bright spot and the second bright spot as the intracellular distance. The average value can be an arithmetic mean value, a geometric mean value, or the like. In the methods of the present invention, when the fluorescence image shown in Fig. 1A is obtained, in the fluorescence image, the distances between the first bright spot and the second bright spot for all combinations of the first bright spot and the second bright spot are measured. More specifically, the distance between the first bright spot (x) and the second bright spot (a), the distance between the first bright spot (x) and the second bright spot (b), and the distance between the first bright spot (x) and the second bright spot (c) are measured. Next, an average value of these distances is calculated, and the obtained average value can be used as the intracellular distance. Similarly, when the fluorescence image shown in Fig. 1B is obtained, an average value of the distance between the first bright spot (x) and the second bright spot (a), the distance between the first bright spot (x) and the second bright spot (b), the distance between the first bright spot (x) and the second bright spot (c), the distance between the first bright spot (y) and the second bright spot (a), the distance between the first bright spot (y) and the second bright spot (b), and the distance between the first bright spot (y) and the second bright spot (c) can be used as the intracellular distance.

In the acquisition method of the present invention, a value regarding the number of monocytes in which the intracellular distance between the first bright spot and the second bright spot is equal to or less than a first threshold value is acquired (or "determined"). As shown in the examples described below, the present inventors have found that blood-derived monocytes from an SMA patient have a smaller ratio of a cell having a short intracellular distance between the first bright spot and the second bright spot, compared to blood-derived monocytes from a healthy subject. Then, the present inventors have found that an SMA patient can be distinguished from a healthy subject based on the number of monocytes in which the intracellular distance between the first bright spot and the second bright spot is equal to or less than the corresponding predetermined threshold value (first threshold value). Therefore, a value regarding the number of monocytes in which the intracellular distance between the first bright spot and the second bright spot is equal to or less than the first threshold value can be an indicator of SMA affection.

The value regarding the number of monocytes in which the intracellular distance between the first bright spot and the second bright spot is equal to or less than the first threshold value is not particularly limited as long as it is a value that can distinguish between an SMA patient and a healthy subject. Examples of such a value include, but are not limited to, the following values in the list below. Hereinafter, the term "satisfy the condition" means that the intracellular distance between the first bright spot and the second bright spot is equal to or less than the first threshold value. The term "does not satisfy the condition" means that the intracellular distance between the first bright spot and the second bright spot is higher than the first threshold value.

- A ratio or a value of the ratio of the number of monocytes that satisfy the condition to the number of all monocytes contained in a measurement sample;
- A ratio or a value of the ratio of the number of monocytes that satisfy the condition to the number of monocytes that contain at least one first bright spot and at least one second bright spot;
- A ratio or a value of the ratio of the number of monocytes that satisfy the condition to the number of monocytes that do not satisfy the condition.

Regarding the ratio of the number of cells, "the ratio of the number of cells B to the number of cells A" is a percentage calculated by [(number of cells B)/(number of cells A)] × 100. Regarding the value of the ratio of the number of cells, the "value of the ratio of the number of cells B to the number of cells A" is a value calculated by (the number of cells B)/(the number of cells A). As the number of all monocytes contained in a measurement sample, the number of monocytes from which a fluorescence image has been acquired may be used.

Preferably, the value for the number of monocytes that satisfy the condition is a ratio or a value of the ratio of the number of monocytes in which the intracellular distance between the first bright spot and the second bright spot is equal to or less than the first threshold value to the number of monocytes that contain at least one first bright spot and at least one second bright spot.

The first threshold value is not particularly limited, but can be appropriately set. For example, the intracellular distances between the first bright spot and the second bright spot in monocytes obtained from a plurality of SMA patients and healthy subjects are acquired. Then, for the intracellular distances, a value that can distinguish between a patient group and a healthy subject group with the highest accuracy is determined, and the value is set as the first threshold value. In setting the threshold value, sensitivity, specificity, a positive predictive value, a negative predictive value and the like can be taken into consideration.

The first threshold value can be set to a value of, for example, 1.2 µm or less, preferably 1.0 µm or less, more preferably 0.9 µm or less. The first threshold value can be set to a value of, for example, 0.5 µm or more, preferably 0.6 µm or more, more preferably 0.7 µm or more. The first threshold value can be, for example, 0.5, 0.6, 0.7, 0.8, 0.9, 1.0, 1.1 or 1.2 µm. In a preferred embodiment, the first threshold value is 0.8 µm.

In the methods of the present invention, a value regarding the number of monocytes that satisfy the condition may be used as an indicator of SMA affection by comparing the value regarding the number of nucleated cells that satisfy the condition with the corresponding predetermined threshold value (second threshold value). When a value regarding the number of monocytes that satisfy the condition is less than the second threshold value, it is suggested that the subject suffers from SMA. When a value regarding the number of monocytes that satisfy the condition is equal to or greater than the second threshold value, it is suggested that the subject does not suffer from SMA.

The second threshold value is not particularly limited, but can be appropriately set. For example, the intracellular distances between the first bright spot and the second bright spot in monocytes obtained from a plurality of SMA patients and healthy subjects are acquired. Then, a value regarding the number of monocytes in which the intracellular distance is equal to or less than the first threshold value is acquired. For this value, a value that can distinguish between a patient group and a healthy subject group with the highest accuracy is determined, and the value is set as the second threshold value. In setting the threshold value, sensitivity, specificity, a positive predictive value, a negative predictive value and the like can be taken into consideration.

When the value regarding the number of monocytes that satisfy the condition is a ratio or a value of the ratio of the number of monocytes that satisfy the condition or a ratio of the number of monocytes that satisfy the condition to the number of monocytes that contain at least one first bright spot and at least one second bright spot, the second threshold value is set between, for example, 10% or more and 18% or less, preferably 11% or more and 15% or less.

A healthcare worker such as a doctor may combine a numerical suggestion for the number of monocytes that satisfy the condition with other information to determine whether a subject suffers from SMA or not. The "other information" includes an evaluation of motor function, a result of genetic testing, and other medical findings.

When the value regarding the number of monocytes that satisfy the condition suggests that a subject suffers from SMA, the subject can be given medical intervention for SMA. Examples of the medical intervention which are not according to the claimed invention include drug administration, surgery, exercise training and physiotherapy. The drug can be appropriately selected from publicly known therapeutic drugs for SMA or drug candidates thereof. Examples of the therapeutic drugs for SMA or candidates thereof include Nusinersen (product name Spinraza (trademark)), Onasemnogene abeparvovec (product name Zolgensma (trademark)) and Risdiplam (product name Evrysdi (trademark)).

Not according to the claimed invention is a method for assisting a determination whether a subject suffers from SMA. In this method, it is determined whether or not a subject suffers from SMA based on the value regarding the number of monocytes that satisfy the condition. When a value regarding the number of monocytes that satisfy the condition is less than the second threshold value, it is determined that the subject suffers from SMA. When a value regarding the number of monocytes that satisfy the condition is equal to or greater than the second threshold value, it is determined that the subject does not suffer from SMA. Based on the value regarding the number of monocytes that satisfy the condition, when it is determined that the subject suffers from SMA, the subject can be given medical intervention for SMA. Details of the medical intervention are as described above.

A further related aspect of the invention relates to a method for acquiring a value that is an indicator of the response of a treatment for SMA as information on SMA. In this method, the subject is an SMA patient who has undergone a treatment for SMA. The treatment for SMA is not particularly limited, but can be appropriately selected from, for example, the above-mentioned medical intervention. The preferred treatment is administration of a drug. Examples of the drug include the above-mentioned Nusinersen, Onasemnogene abeparvovec and Risdiplam. The treatment for SMA may be the first treatment for the subject or the second or subsequent treatment.

In the method according to this aspect of the invention, a sample prepared from a blood specimen obtained from an SMA patient who has undergone a treatment for SMA is used as a measurement sample. The time for collecting blood from the patient is not particularly limited as long as it is after receiving a treatment for SMA, but for example, the time is at a time point when 0.5 hours, 1 hour, 2 hours, 3 hours, 4 hours, 5 hours, 6 hours, 7 hours, 8 hours, 9 hours, 10 hours, 12 hours, 15 hours, 18 hours, 1 day, 2 days, 3 days, 4 days, 5 days, 6 days, 7 days, 8 days, 9 days, 10 days, 11 days, 12 days, 13 days, 2 weeks, 3 weeks, 4 weeks or 1 month have elapsed from receiving a treatment for SMA. In the method according to this aspect of the invention, the measurement sample can be obtained in the same manner as in the acquisition method of the invention described above, except that a blood specimen obtained from an SMA patient who has undergone a treatment for SMA is used.

In the method according to this aspect of the invention, except that a measurement sample from an SMA patient who has undergone a treatment for SMA is used, it is possible to acquire a fluorescence image of a monocyte in the measurement sample, to acquire the intracellular distance between the first bright spot and the second bright spot in the fluorescence image, and to acquire a value regarding the number of monocytes in which the intracellular distance is equal to or less than the first threshold value, in the same manner as in the acquisition method of the above-mentioned methods of the invention. The first and second bright spots, intracellular distance, and value regarding the number of monocytes in which the intracellular distance is equal to or less than the first threshold value are as described above.

In the methods according to this aspect of the invention, a value regarding the number of monocytes that satisfy the condition can be used as an indicator of the response of a treatment for SMA by comparing the value regarding the number of monocytes that satisfy the condition with the second threshold value. The second threshold is as described above. As shown in the examples described below, the present inventors have found that when a treatment for SMA is successful, blood-derived monocytes collected after the treatment have an increased ratio of a cell having a shorter intracellular distance between the first bright spot and the second bright spot, compared to blood-derived monocytes collected before the treatment. Then, the present inventors have found that the response of a treatment for SMA can be evaluated based on the number of monocytes in which the intracellular distance between the first bright spot and the second bright spot is equal to or less than the first threshold value. Therefore, the value regarding the number of monocytes that satisfy the condition can be an indicator of the response of a treatment for SMA.

When a value regarding the number of monocytes that satisfy the condition is below the second threshold value, it is suggested that the treatment for SMA is not successful in the patient. When a value regarding the number of monocytes that satisfy the condition is equal to or greater than the second threshold value, it is suggested that the treatment for SMA is successful in the patient. The second threshold is as described above.

In a further embodiment of this aspect of the invention, it is determined whether or not the treatment for SMA is successful in the patient based on the value regarding the number of monocytes that satisfy the condition. When a value regarding the number of monocytes that satisfy the condition is below the second threshold value, it can be determined that the treatment for SMA is not successful in the patient. When a value regarding the number of monocytes that satisfy the condition is equal to or higher than the second threshold value, it can be determined that the treatment for SMA is successful in the patient.

A further related aspect of the invention relates to a method for acquiring a value that is an indicator of the response of a treatment for SMA as information on SMA by comparing the analysis results of a measurement sample before receiving a treatment for SMA and a measurement sample after receiving the treatment from the same SMA patient. In this aspect of the invention, the subject is an SMA patient. As the measurement samples, a measurement sample derived from a blood specimen obtained from a patient before receiving a treatment for SMA (hereinafter, also referred to as "measurement sample before treatment") and a measurement sample derived from a blood specimen obtained from the patient after receiving the treatment for SMA (hereinafter, also referred to as "measurement sample after treatment") are used.

The treatment for SMA is not particularly limited, but can be appropriately selected from, for example, the above-mentioned medical intervention. The treatment for SMA may be a one-time treatment or may be a treatment performed multiple times over a certain period of time. Examples of the one-time treatment include single drug administration and surgery. Examples of the treatment performed multiple times over a certain period of time include continuous drug administration, physiotherapy and exercise training. The preferred treatment is administration of a drug. Examples of the drug include the above-mentioned Nusinersen, Onasemnogene abeparvovec and Risdiplam.

In the method according to this aspect of the invention, the "patient before receiving a treatment for SMA" includes not only an SMA patient who has never undergone the treatment, but also an SMA patient who has undergone the treatment in the past. For example, when a treatment in which a drug is administered twice at different times is performed, at the time of receiving the first drug administration, the patient corresponds to a patient before receiving the treatment of the second drug administration.

The time for collecting blood from a patient before receiving the treatment for SMA is not particularly limited as long as it is before receiving the treatment. Preferably, the time is the day before the day of the treatment for SMA or the same day as the day of the treatment. The time for collecting blood from a patient after receiving the treatment for SMA is as described above. In the method according to this aspect of the invention, a measurement sample may be prepared and analyzed each time a blood specimen is collected. Alternatively, collected blood specimens may be stored, and at a later date, measurement samples before and after the treatment may be prepared from each of the blood specimens and sequentially analyzed.

In the method according to this aspect of the invention, a fluorescence image of a monocyte contained in each of the measurement sample before the treatment and the measurement sample after the treatment is acquired, the intracellular distance between the first bright spot and the second bright spot in the fluorescence image is acquired, and a value regarding the number of monocytes in which the intracellular distance is equal to or less than the first threshold value is acquired, in the same manner as in the acquisition method of the above-mentioned methods of the invention. The first and second bright spots, intracellular distance, and value regarding the number of monocytes in which the intracellular distance is equal to or less than the first threshold value are as described above.

In the method according to this aspect of the invention, a value regarding the number of monocytes that satisfy the condition acquired from the measurement sample before the treatment and a value regarding the number of monocytes that satisfy the condition acquired from the measurement sample after the treatment are obtained. In this method, these two values can be used as an indicator of the response of a treatment for SMA.

When value regarding the number of monocytes that satisfy the condition acquired from the measurement sample after the treatment is less than a value regarding the number of monocytes that satisfy the condition acquired from the measurement sample before the treatment, it is suggested that the treatment for SMA is not successful in the patient. When a value regarding the number of monocytes that satisfy the condition acquired from the measurement sample after the treatment is equal to or greater than a value regarding the number of monocytes that satisfy the condition acquired from the measurement sample before the treatment, or equal to or greater than the second threshold value, it is suggested that the treatment for SMA is successful in the patient.

In a further embodiment, it is determined whether or not the treatment for SMA is successful in the patient based on the values regarding the number of monocytes that satisfy the condition acquired from the two measurement samples. When a value regarding the number of monocytes that satisfy the condition acquired from the measurement sample after the treatment is less than a value regarding the number of monocytes that satisfy the condition acquired from the measurement sample before the treatment, it is determined that the treatment for SMA is not successful in the patient. When a value regarding the number of monocytes that satisfy the condition acquired from the measurement sample after the treatment is equal to or greater than a value regarding the number of monocytes that satisfy the condition acquired from the measurement sample before the treatment, or equal to or greater than the second threshold value, it can be determined that the treatment for SMA is successful in the patient.

When it is suggested or determined that the treatment for SMA is successful in the patient, the treatment to the patient can be continued. On the other hand, when it is suggested or determined that the treatment for SMA is not successful in the patient, a doctor or the like can consider, for example, changing the dose or type of drug, changing the type of treatment, or adding a treatment.

Not according to the claimed invention is a reagent kit for acquiring information on SMA. The reagent kit includes a substance capable of specifically binding to an SMN protein, a substance capable of specifically binding to coilin in a monocyte, a first fluorescent dye and a second fluorescent dye. The reagent kit can be used in the methods of the present invention for acquiring a value that is an indicator of SMA affection or a value that is an indicator of the response of a treatment for SMA. The substance capable of specifically binding to each of the proteins and the first and second fluorescent dyes are as described above.

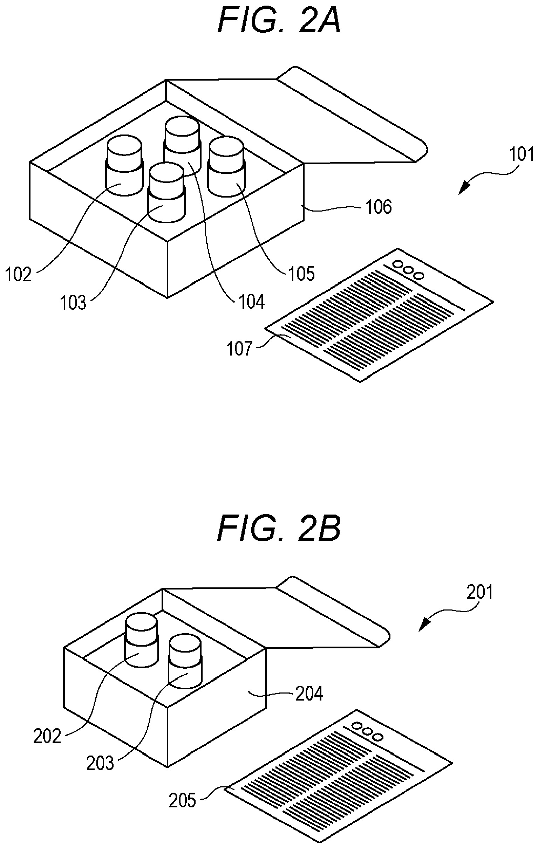

The reagent kit in a box in which a container containing each reagent is packed may be provided to the user. The box may include a package insert. The package insert may describe the configuration of the reagent kit, the composition of each reagent, the method of use, or the like. An example of the reagent kit is shown in Fig. 2A. In Fig. 2A, reference numbers 101 represents the reagent kit, 102 represents a container containing a reagent containing a substance capable of specifically binding to an SMN protein, 103 represents a container containing a reagent containing a substance capable of specifically binding to coilin in a monocyte, 104 represents a container containing a reagent containing the first fluorescent dye, 105 represents a container containing a reagent containing the second fluorescent dye, 106 represents a packing box, and 107 represents the package insert. In this example, when the substance capable of specifically binding to an SMN protein is an antibody, the reagent containing the first fluorescent dye may be a secondary antibody labeled with the first fluorescent dye. When the substance capable of specifically binding to coilin in a monocyte is an antibody, the reagent containing the second fluorescent dye may be a secondary antibody labeled with the second fluorescent dye.

In one example, the substance capable of specifically binding to an SMN protein may be labeled with the first fluorescent dye. The substance capable of specifically bind to coilin in a monocyte may be labeled with the second fluorescent dye. An example of the reagent kit according to this example is shown in Fig. 2B. In Fig. 2B, reference numbers 201 represents a reagent kit, 202 represents a container containing a reagent that contains a substance capable of specifically binding to an SMN protein and is labeled with the first fluorescent dye, 203 represents a container that contains a reagent containing a substance capable of specifically binding to coilin in a monocyte and is labeled with the second fluorescent dye, 204 represents a packing box, and 205 represents the package insert.

The reagent kit may further include a reagent containing a substance capable of specifically binding to a surface antigen marker, and a reagent containing the third fluorescent dye. When the substance capable of specifically binding to a surface antigen marker is an antibody, the reagent containing the third fluorescent dye may be a secondary antibody labeled with the third fluorescent dye. Alternatively, the substance capable of specifically binding to a surface antigen marker may be labeled with the third fluorescent dye. The reagent kit may further include a reagent containing a dye for nuclear staining.

Not according to the claimed invention is a use of reagents for manufacturing a reagent kit for acquiring information on SMA, in which the reagents are a reagent containing a substance capable of specifically binding to an SMN protein, a reagent containing a substance capable of specifically binding to coilin in a monocyte, a reagent containing the first fluorescent dye, and a reagent containing the second fluorescent dye. Also not according to the claimed invention is a use of reagents for manufacturing a reagent kit for acquiring information on SMA, in which the reagents are a reagent that contains a substance capable of specifically binding to an SMN protein and is labeled with the first fluorescent dye, and a reagent that contains a substance capable of specifically binding to coilin in a monocyte and is labeled with the second fluorescent dye.

Not according to the claimed invention is an acquisition device for information on SMA.

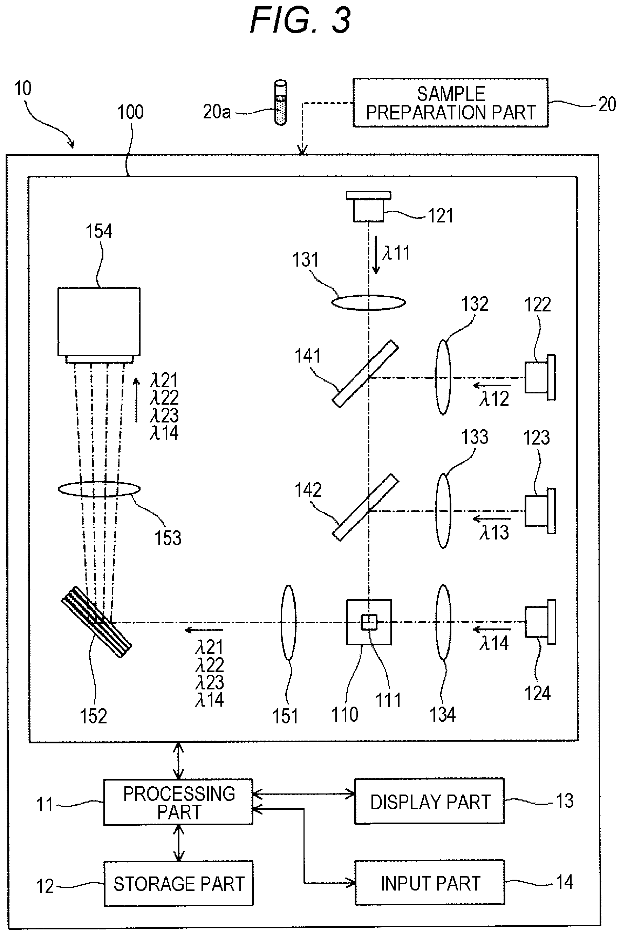

A description is made of an example of the acquisition device with reference to the drawings. The acquisition device 10 shown in Fig. 3 is a device having an IFC configuration including a camera and a flow cell, but the acquisition device is not limited to the form exemplified in Fig. 3. The acquisition device may be a device including an eyepiece lens and an objective lens, and having a configuration of a fluorescence microscope for observing a cell on a slide and acquiring a fluorescence image of the cell.

The acquisition device 10 shown in Fig. 3 includes an imaging unit 100 and a processing part 11. The imaging unit 100 acquires a fluorescence image of a monocyte containing an SMN protein labeled with the first fluorescent dye and coilin labeled with the second fluorescent dye. The processing part 11 analyzes the acquired fluorescence image. The acquisition device 10 shown in Fig. 3 includes a storage part 12, a display part 13 and an input part 14 connected to the processing part 11.

In the example of Fig. 3, the imaging unit 100 includes light sources 121 to 124 and an imaging part 154. The light sources 121 to 124 irradiate a monocyte stained with a fluorescent dye with light. The imaging part 154 uses a camera such as a CCD (Charge-Coupled Device) camera or a TDI (Time Delay Integration) camera. The imaging unit 100 includes condenser lenses 131 to 134, 151 and 153, dichroic mirrors 141 and 142, and an optical unit 152. Fluorescence images corresponding to the respective three types of fluorescent dyes and a transmitted light image are acquired by the four types of light sources 121 to 124 provided in the imaging unit 100. The three types of fluorescent dyes may be the above-mentioned first, second and third fluorescent dyes. Alternatively, the above-mentioned first and second fluorescent dyes and the above-mentioned dye for nuclear staining may be used. The transmitted light image is also referred to as a bright field image.

The acquisition device 10 shown in Fig. 3 acquires a fluorescence image of a monocyte in a measurement sample 20a prepared by a sample preparation part 20. The sample preparation part 20 prepares the measurement sample 20a from a blood specimen of a subject. The sample preparation part 20 supplies the measurement sample 20a to the imaging unit 100. The sample preparation part 20 includes a mixing container for mixing the blood specimen and the reagent, a dispensing unit for dispensing the blood specimen and the reagent into the mixing container, a heating unit for heating the mixing container, and the like. The sample preparation part 20 may be a device separate from the acquisition device 10 or may be provided in the acquisition device 10. When the acquisition device 10 includes the sample preparation part 20, the sample preparation part 20 is connected to the processing part 11. In addition, the sample preparation part 20 is configured to be controllable by the processing part 11.

The acquisition device 10 shown in Fig. 3 includes a flow cell 110 for flowing the measurement sample 20a. The flow cell 110 is made of a translucent resin or glass. The flow cell 110 has a flow path 111 for flowing the measurement sample 20a. The flow cell 110 is provided in the imaging unit 100. In the imaging unit 100, the light sources 121 to 124 are configured to irradiate the flow cell 110 with light. The imaging part 154 is configured to acquire a fluorescence image of a cell flowing through the flow path 111 of the flow cell 110.

As described above, the imaging unit 100 is configured such that the measurement sample 20a flowing through the flow path 111 of the flow cell 110 is irradiated with light emitted from the light sources 121 to 124. Each of the light sources 121 to 123 can be, for example, a semiconductor laser light source, and the light source 124 can be, for example, a white LED. The light source 121 is a light source for exciting the first fluorescent dye. The light source 121 emits laser light including light having a wavelength of λ11. The light source 122 is a light source for exciting the second fluorescent dye. The light source 122 emits laser light including light having a wavelength of λ12. The light source 123 is a light source for exciting the third fluorescent dye or the dye for nuclear staining. The light source 123 emits laser light including light having a wavelength of λ13. The light source 124 emits white light having a wavelength of λ14 that passes through a cell.

In the example of Fig. 3, the condenser lenses 131 to 134 are arranged between the light sources 121 to 124 and the flow cell 110, respectively, and light emitted from the light sources 121 to 124 is focused on the flow cell 110. The dichroic mirror 141 transmits light having a wavelength of λ11. The dichroic mirror 141 reflects light having a wavelength of λ12. The dichroic mirror 142 transmits light having wavelengths of λ11 and λ12. The dichroic mirror 142 reflects light having a wavelength of λ13. Such an optical system allows the flow path 111 of the flow cell 110 to be irradiated with light from the light sources 121 to 124. When the measurement sample 20a flowing through the flow path 111 is irradiated with light having wavelengths of λ11 to λ13, the fluorescent dye that labels a cell fluoresces.

Specifically, when the first fluorescent dye that labels an SMN protein is irradiated with light having a wavelength of λ11, the first fluorescent dye produces a first fluorescence having a wavelength of λ21. When the second fluorescent dye that labels a given nuclear protein (e.g., coilin) is irradiated with light having a wavelength of λ12, the second fluorescent dye produces a second fluorescence having a wavelength of λ22. When the third fluorescent dye that labels a surface antigen marker or the dye for nuclear staining that labels a cell nucleus is irradiated with light having a wavelength of λ13, the third fluorescent dye or the dye for nuclear staining emits a third fluorescence having a wavelength of λ23. When the sample 20a is irradiated with white light from the light source 124, the white light passes through a cell so that a bright field image is obtained.

In the example of Fig. 3, the condenser lens 151, the optical unit 152, and the condenser lens 153 are arranged in this order between the flow cell 110 and the imaging part 154 along the optical path of the laser beam from the flow cell 110 side. The condenser lens 151 focuses the first to third fluorescences generated from the measurement sample 20a and transmitted light passing through the measurement sample 20a on the optical unit 152. The optical unit 152 is configured by, for example, stacking four dichroic mirrors. The four dichroic mirrors reflect the first to third fluorescences at different angles from each other. The four dichroic mirrors separate them on the light receiving surface of the imaging part 154. The condenser lens 153 focuses light reflected by the optical unit 152 on the light receiving surface of the imaging part 154.

The imaging part 154 captures an image formed of the first to third fluorescences and the transmitted light to acquire three types of fluorescence images corresponding to the respective first to third fluorescences and a bright field image corresponding to the transmitted light and transmit the acquired images to the processing part 11. The fluorescence images corresponding to the first to third fluorescences are also referred to as "a first image", "a second image" and "a third image", respectively. The processing part 11 corrects each image by software such that the positional relationships of the object and a pixel match between the first to third images and the bright field image transmitted from the imaging part 154. In order to analyze the intracellular distance between the first bright spot and the second bright spot, the first image and the second image are the same size as each other.

The imaging unit 100 further includes a light receiving part arranged so as to detect forward scattered light emitted from individual particles in the measurement sample 20a, and a light receiving part arranged so as to detect side scattered light emitted from individual particles in the measurement sample 20a. The side scattered light is not limited to light scattered in the direction of 90° with respect to the optical axis direction of the light source, but is, for example, light scattered in the direction of 80° or more and 100° or less with respect to the optical axis direction. The forward scattered light is not limited to light scattered in the optical axis direction of the light source, but may be, for example, light scattered in the direction of -10° or more and 10° or less with respect to the optical axis direction. Each light receiving part transmits a forward scattered light signal and a side scattered light signal according to the light receiving level to the processing part 11.

The processing part 11 extracts a fluorescence image of a monocyte from the fluorescence image captured based on optical information such as scattered light signals emitted from individual particles in the measurement sample 20a. By executing software stored in the storage part 12 described below, the fluorescence image of a monocyte is analyzed in the imaging unit 100 to acquire the intracellular distance between the first bright spot and the second bright spot, and then acquire a value regarding the number of monocytes in which the intracellular distance is equal to or less than the first threshold value. The processing part 11 is composed of a CPU. The processing part 11 executes arithmetic processing related to processing and analysis of the fluorescence image. The processing part 11 executes various processes including image analysis of the fluorescence image based on a computer program stored in the storage part 12. The processing part 11 is connected to the imaging unit 100, the storage part 12, the display part 13 and the input part 14. The processing part 11 receives a signal from each part to acquire various information. The processing part 11 outputs a control signal to each part to control each part.

The storage part 12 is composed of a RAM, a ROM, a solid state drive (SSD), a hard disk, or the like. The storage part 12 stores a computer program executed by the processing part 11 for analysis of the fluorescence image. The display part 13 is composed of a display. The display part 13 displays the fluorescence image of a cell, a value regarding the number of monocytes in which the intracellular distance is equal to or less than the first threshold value, auxiliary information for assisting visual analysis, or the like. The input part 14 is composed of a mouse and a keyboard. The input part 14 is used for inputting information such as a specimen ID, switching display screens, and selecting the fluorescence image, etc. The configuration of the storage part 12, the display part 13 and the input part 14 is not particularly limited. Instead of the display part 13 and the input part 14, a touch panel in which an input part is arranged on the surface of the display part may be provided as a display input part. Examples of the touch panel include a touch panel of a well-known type such as a capacitance type.

By executing software stored in the storage unit 12, the processing part 11 processes the first and second images captured by the imaging part 154 to extract the first and second bright spots from the first and second images, respectively. When a monocyte is labeled with the dye for nuclear staining, the processing part 11 extracts a nuclear region from the third image. The processing part 11 may display the fluorescence image on the display part 13 for each cell.

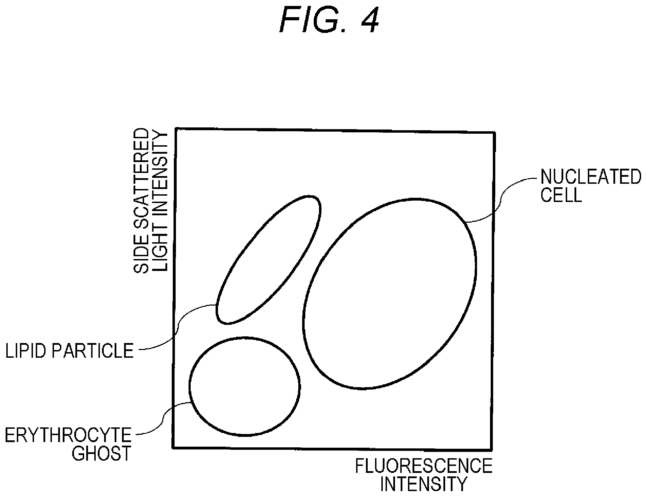

When a monocyte is labeled with the third fluorescent dye, the processing part 11 uses data of the signal intensity of the third fluorescence and data of the forward scattered light intensity or the side scattered light intensity to create a 2D scattergram. For example, when a 2D scattergram with the fluorescence intensity on the X-axis and the side scattered light intensity on the Y-axis is created, as shown in Fig. 4, a monocyte labeled with the third fluorescent dye is distributed to form a subpopulation. The number of cells in the subpopulation of monocyte can be acquired, for example, by counting the cell in the subpopulation on the 2D scattergram by analysis software stored in the storage part 12. Fig. 4 is an example of the 2D scattergram. The processing part 11 extracts the first and second images of a monocyte labeled with the third fluorescent dye, and then extracts the first and second bright spots from the fluorescence images.

The extraction of the first and second bright spots by the processing part 11 is performed, for example, as follows. The processing part 11 sets a threshold value of the pixel value that is the boundary between the bright spot and the background based on the pixel value of each pixel constituting the first image. The method itself for setting a threshold value for binarization processing of an image is publicly known, and examples thereof include a mode method and a P-tile method. The processing part 11 binarizes the first image based on whether or not the pixel value of each pixel constituting the first image is higher than the threshold value. Then, the processing part 11 extracts a range in which a pixel having a pixel value higher than the threshold value is distributed as the first bright spot. The processing part 11 also performs binarization processing on the second image and extraction for a second bright spot in the same manner.

The processing part 11 may perform noise removal processing of the fluorescence image before the binarization processing. The noise removal processing of an image is performed using a noise removal means such as a top hat filter.

When a monocyte is labeled with the dye for nuclear staining, the processing part 11 also performs the binarization processing on the third image in the same manner, and extract a range in which a pixel having a pixel value higher than the threshold value is distributed as a nuclear region. A monocyte in which the first and second bright spots extracted from the first and second images are outside the nuclear region is excluded from the analysis.

The processing part 11 acquires the intracellular distance between a first bright spot and a second bright spot extracted by binarization processing. When there is a plurality of first bright spot and/or second bright spot in one monocyte, among those bright spots, the processing part 11 extracts the closest first bright spot and the second bright spot to acquire the intracellular distance between the extracted first bright spot and the second bright spot. The closest first bright spot and the second bright spot are extracted, for example, based on the center of gravity coordinate of each bright spot. Alternatively, the intracellular distances between all combinations of the first bright spot and the second bright spot are measured, and the combination of a first bright spot and a second bright spot having the shortest distance is extracted as the closest first bright spot and the second bright spot.

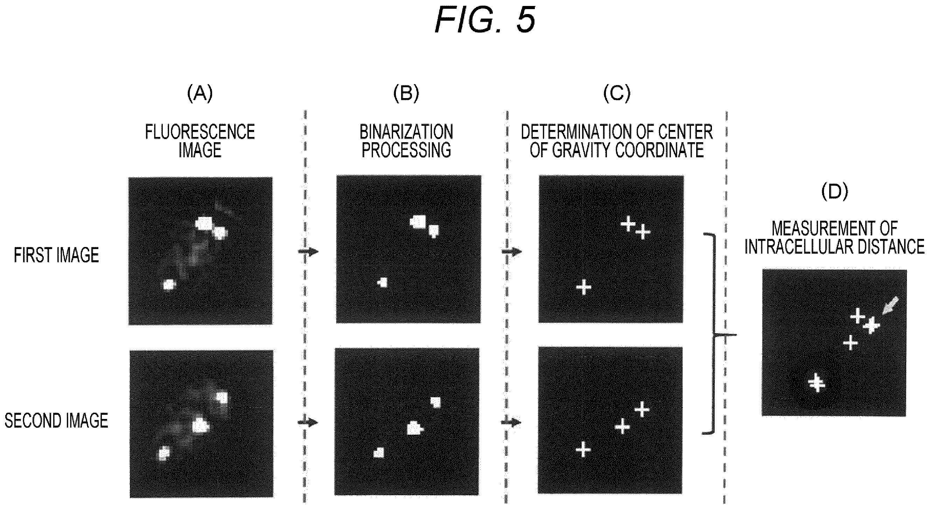

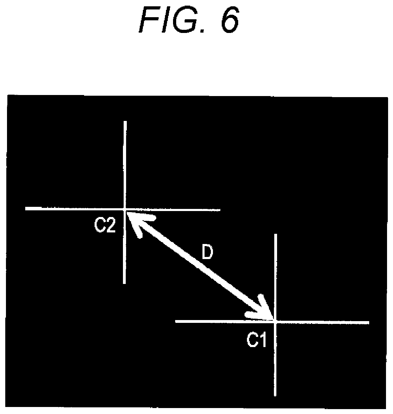

As an example, a description is made of acquisition of the distance D between the centers of gravity of the first bright spot and the second bright spot with reference to Fig. 5. Fig. 5A shows the first image and the second image obtained by the imaging part 154. The processing part 11 binarizes each image as described above to extract a first bright spot and a second bright spot as shown in Fig. 5B. The processing part 11 calculates the center of gravity coordinates of the extracted first bright spot and the second bright spot to determine the centers of gravity of the first bright spot and the second bright spot as shown in Fig. 5C. The method itself for calculating the coordinate of the center of gravity of a predetermined region in an image is publicly known, and the coordinate can be calculated by a predetermined formula or the like. The processing part 11 acquires the distance between the centers of gravity of the closest first bright spot and the second bright spot. Fig. 5D shows a superposed image of the first image and the second image. In this example, the processing part 11 extracts a first bright spot and a second bright spot indicated by the arrow as the closest first bright spot and the second bright spot to acquire the distance D between the centers of gravity. Specifically, the processing part 11 acquires the distances between the centers of gravity for all combinations of the first bright spot and the second bright spot. For example, when the first image has first bright spots (1), (2) and (3), and the second image has second bright spots (i), (ii) and (iii), the distance between the centers of gravity of the first bright spot (1) and the second bright spot (i), the distance between the centers of gravity of the first bright spot (1) and the second bright spot (ii), the distance between the centers of gravity of the first bright spot (1) and the second bright spot (iii), the distance between the centers of gravity of the first bright spot (2) and the second bright spot (i), the distance between the centers of gravity of the first bright spot (2) and the second bright spot (ii), the distance between the centers of gravity of the first bright spot (2) and the second bright spot (iii), the distance between the centers of gravity of the first bright spot (3) and the second bright spot (i), the distance between the centers of gravity of the first bright spot (3) and the second bright spot (ii), and the distance between the centers of gravity of the first bright spot (3) and the second bright spot (iii) are acquired. Next, the processing part 11 compares these distances between the centers of gravity, and sets the shortest distance between the centers of gravity as the distance D between the centers of gravity. The distance between the centers of gravity can be a distance between the center of gravity coordinate C1 of the first bright spot and the center of gravity coordinate C2 of the second bright spot, as shown in Fig. 6. Then, the processing part 11 compares the distance D between the centers of gravity with the first threshold value. Although Fig. 5D illustrates that, for convenience of description, the distance between the centers of gravity is acquired in the superimposed image, when there are data on the center of gravity coordinates of the respective bright spots in the first and second images, it is not essential for the processing by the processing part 11 to create a superimposed image.

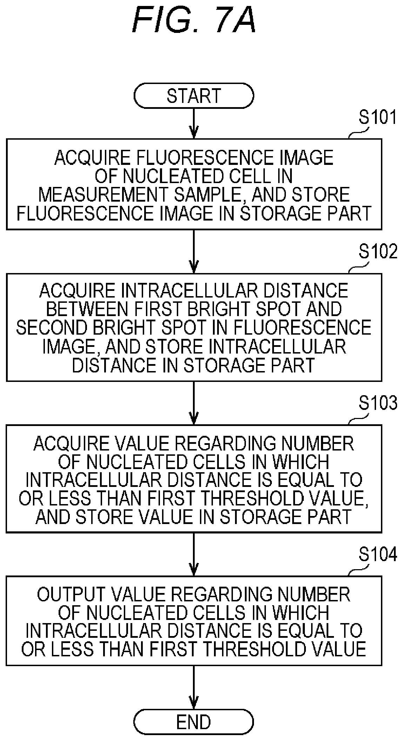

A description is made of the processing procedure executed by the acquisition device 10 with reference to the drawings. With reference to Fig. 7A, a description is made of a processing procedure for acquiring and outputting a value regarding the number of monocytes in which the intracellular distance between the first bright spot and the second bright spot is equal to or less than the first threshold value. In step S101, the processing part 11 controls the fluid circuit of the acquisition device 10 to flow the measurement sample 20a into the flow cell 110. The processing part 11 causes the light sources 121 to 124 to emit light. As a result, each cell in the measurement sample 20a flowing through the flow cell 110 is irradiated with light. The processing part 11 causes the imaging part 154 to capture a fluorescence image and a bright-field image of a cell. As a result, the fluorescence image and the bright-field image are acquired for each cell. As the fluorescence image, a first image corresponding to the first fluorescent dye and a second image corresponding to the second fluorescent dye are acquired. The processing part 11 stores the fluorescence image and the bright field image for each cell in the storage part 12. The fluorescence image and bright-field image stored in the storage part 12 include a fluorescence image and a bright-field image of a monocyte.

In step S 102, the processing part 11 binarizes and analyzes the first image and the second image to extract the first bright spot and the second bright spot in a monocyte in the fluorescence image as described above. Then, the processing part 11 determines the center of gravity coordinates of the first bright spot and the second bright spot to acquire a distance between the centers of gravity as the intracellular distance. When there is a plurality of first bright spot and/or second bright spot in a monocyte, the processing part 11 determines the center of gravity coordinate of each bright spot, calculates the distance between the centers of gravity, and acquires the closest distance between the centers of gravity among the respective distances between the centers of gravity as the intracellular distance. The processing part 11 stores the intracellular distance for each cell in the storage part 12. In step S103, the processing part 11 compares the intracellular distance with the first threshold for each monocyte. Then, the processing part 11 counts the number of monocytes in which the intracellular distance is equal to or less than the first threshold, acquires a value regarding the number of monocytes in which the intracellular distance is equal to or less than the first threshold, and stores the value in the storage part 12. The value regarding the number of monocytes in which the intracellular distance is equal to or less than the first threshold value is as described above.

In step S104, the processing part 11 outputs a value regarding the number of monocytes in which the intracellular distance is equal to or less than the first threshold value. For example, the processing part 11 displays the value on the display part 13, prints the value with a printer, or transmits the value to a mobile device. When outputting the value, the number itself of monocytes in which the intracellular distance is equal to or less than the first threshold value is also output as reference information. The above-mentioned second threshold value is output as reference information. As described above, the acquisition device can provide a doctor or the like with a value regarding the number of monocytes in which the intracellular distance is equal to or less than the first threshold value as information on SMA. As described above, the value is an indicator of SMA affection or an indicator of the response of a treatment for SMA.

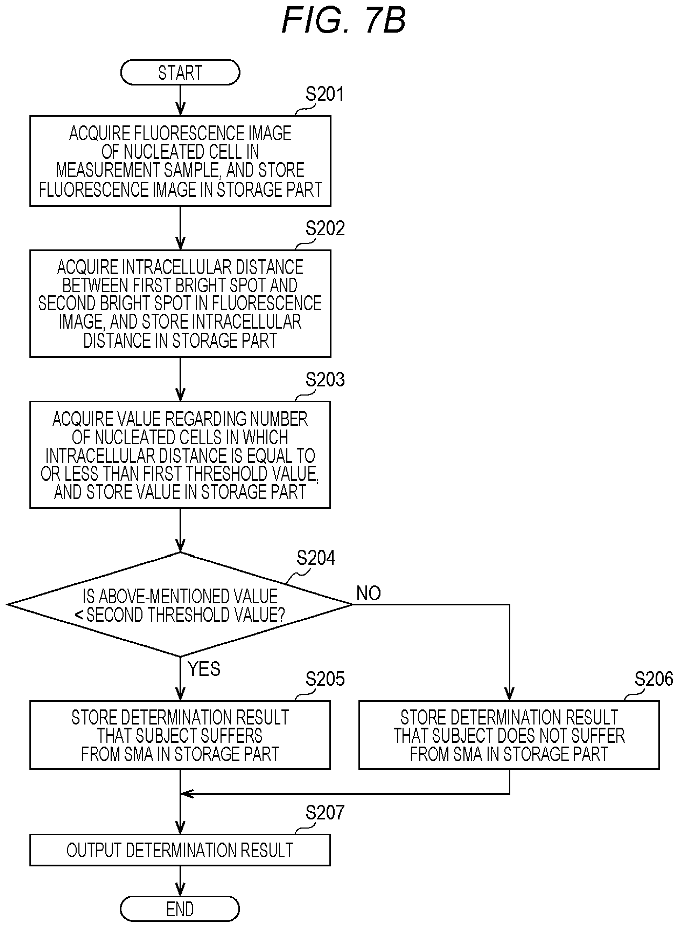

With reference to Fig. 7B, a description is made of a flow for determining whether or not a subject suffers from SMA based on a value regarding the number of monocytes in which the intracellular distance is equal to or less than the first threshold value. In step S201, the processing part 11 captures a fluorescence image of a monocyte in the measurement sample 20a with the acquisition device 10 by the same method as in step S 101 to acquire the first image and the second image. The processing part 11 stores the fluorescence image and the bright field image for each cell in the storage part 12. In step S202, the processing part 11 extracts the first bright spot and the second bright spot in a monocyte in the fluorescence image by the same method as in step S 102 to acquire the intracellular distance between the first bright spot and the second bright spot. The processing part 11 stores the intracellular distance for each cell in the storage part 12. In step S203, the processing part 11 acquires a value regarding the number of monocytes in which the intracellular distance is equal to or less than the first threshold value by the same method as in step S103, and stores the value in the storage part 12.

In step S204, the processing part 11 compares the acquired value with the second threshold value. In step S204, when the acquired value is less than the second threshold value, the process proceeds to step S205. In step S205, the processing part 11 stores a determination result that the subject suffers from SMA in the storage part. In step S204, when the acquired value is equal to or greater than the second threshold value, the process proceeds to step S206. In step S206, the processing part 11 stores a determination result that the subject does not suffer from SMA in the storage part. In step S207, the processing part 11 outputs the determination result. For example, the processing part 11 displays the determination result on the display part 13, prints the determination result with a printer, or transmits the determination result to a mobile device. As described above, the acquisition device can provide a doctor or the like with a determination result of whether or not a subject suffers from SMA.

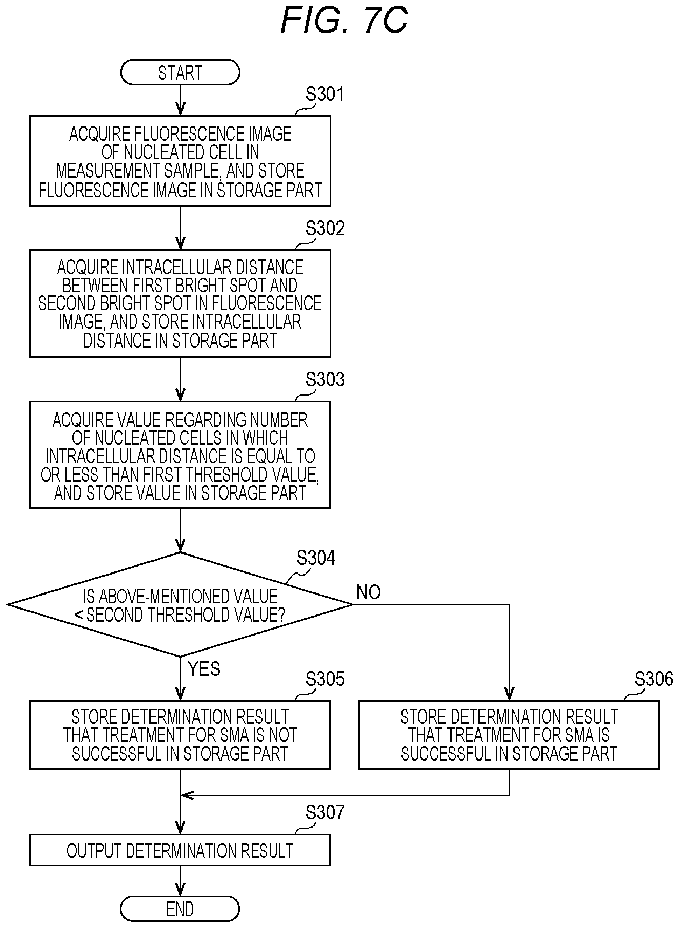

With reference to Fig. 7C, a description is made of the flow for determining whether or not a treatment for SMA is successful in an SMA patient based on a value regarding the number of monocytes in which the intracellular distance is equal to or less than the first threshold value. In step S301, the processing part 11 captures a fluorescence image of a monocyte in the measurement sample 20a with the acquisition device 10 by the same method as in step S 101 to acquire the first image and the second image. The processing part 11 stores the fluorescence image and the bright field image for each cell in the storage part 12. In step S302, the processing part 11 extracts the first bright spot and the second bright spot in a monocyte in the fluorescence image by the same method as in step S 102 to acquire the intracellular distance between the first bright spot and the second bright spot. The processing part 11 stores the intracellular distance for each cell in the storage part 12. In step S303, the processing part 11 acquires a value regarding the number of monocytes in which the intracellular distance is equal to or less than the first threshold value by the same method as in step S103, and stores the value in the storage part 12.

In step S304, the processing part 11 compares the acquired value with the second threshold value. In step S304, when the acquired value is less than the second threshold value, the process proceeds to step S305. In step S305, the processing part 11 stores a determination result that a treatment for SMA is not successful in a patient in the storage part. In step S304, when the acquired value is equal to or greater than the second threshold value, the process proceeds to step S306. In step S306, the processing part 11 stores a determination result that a treatment for SMA is successful in a patient in the storage part. In step S307, the processing part 11 outputs the determination result. For example, the processing part 11 displays the determination result on the display part 13, prints the determination result with a printer, or transmits the determination result to a mobile device. As described above, the acquisition device can provide a doctor or the like with a determination result of whether or not a treatment for SMA is successful in an SMA patient.

Hereinafter, a detailed description is made of the present invention with reference to Examples, but the present invention is not limited to the Examples.

### EXAMPLES

### Example 1: Distinguishing between healthy subject and SMA patient

### (1) Preparation of measurement sample

### (1.1) Monocyte labeling, hemolysis treating and nucleated cell immobilizing

Peripheral blood collected from each of 2 healthy subjects and 10 SMA patients into a heparin-containing blood collection tube was used as a specimen. Into a 15 mL conical tube, 1.5 mL of peripheral blood was transferred, and then 30 µL of Clear Back (MTG-001, MBL), a human Fc receptor blocking reagent, was added, followed by incubation in the dark for 15 minutes. Into this, 10 µL of a solution of PerCP/Cy5.5 (trademark)-labeled anti-human CD33 monoclonal antibody (WM53, BioLegend) was added, followed by incubation in the dark for 30 minutes. CD33 is a monocyte surface antigen marker. 10 mL of BD Phosflow (trademark) Lyse/Fix Buffer (BD Biosciences), which had been preheated to 37°C, was further added, followed by incubation at 37°C for 10 minutes. As a result, an erythrocyte in peripheral blood was hemolyzed, and a nucleated cell was immobilized. The tube was centrifuged at 300 × g for 5 minutes to remove the supernatant, and the cell was washed with 15 mL of PBS (-).

### (1.2) Immunostaining of SMN protein and coilin in nucleated cell

To a cell immobilized in the above-mentioned (1.1), 1 mL of 0.2% Triton (trademark) X-100/1% BSA/PBS (-) was added for suspension, followed by incubation in the dark at room temperature for 5 minutes. Into this, 3 mL of 1% BSA/Perm I (BD Biosciences) was added, followed by incubation in the dark at room temperature for 60 minutes. The tube was centrifuged at 200 × g for 10 minutes to remove the supernatant, and the cell was washed twice with 1.5 mL of 1% BSA/Perm I. To the cell, 100 µL of 0.05% Tween (trademark) 20/1% BSA/Perm I (hereinafter, also referred to as "staining/washing buffer") was added to suspend the cell. Then, the cell suspension was transferred into a 1.5 mL microtube. Then, 5 µL of the cell suspension was taken and diluted with 195 µL PBS (-) to count the cell. The cell suspension (1 × 10⁶ cells/50 µL) was placed in each of the two microtubes. To one microtube were added 1 µg of Alexa Fluor (registered trademark) 488-labeled anti-SMN monoclonal antibody (2B1, Novus Biologicals) and 0.2 µg of rabbit anti-human coilin antibody (10967-1-AP, Proteintech), whereas to the other microtube were added 1 µg of Alexa Fluor (registered trademark) 488-labeled mouse isotype control (MOPC21, IgG1, BioLegend) and 0.2 µg of rabbit isotype control (10967-1-AP, rabbit IgG, Proteintech), followed by incubation in the dark at 4°C for 45 minutes. To this, 600 µL of staining/washing buffer was added, followed by centrifugation at 500 × g for 5 minutes to remove the supernatant. The cell was washed by performing the same operation twice more. Alexa Fluor (registered trademark) 405-labeled anti-rabbit IgG antibody was added to each microtube, followed by incubation in the dark at 4°C for 15 minutes. In the same manner as described above, the cell was washed twice with the staining/washing buffer. The cell was further washed with 500 µL of PBS (-) and then suspended in 50 µL of PBS (-). As a result, a measurement sample containing a nucleated cell in which an SMN protein and coilin were immunostained was obtained.

### (2) Cell measuring and image analyzing

### (2.1) Extraction of monocyte fraction

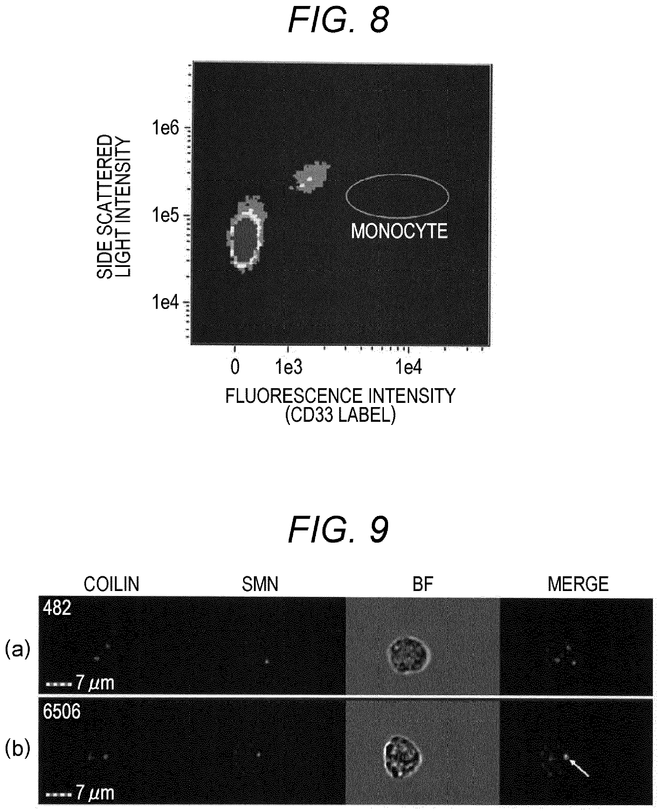

The cell obtained in the above-mentioned (1.2) was measured and imaged using an imaging flow cytometer MI-1000 (Sysmex Corporation) to acquire an optical signal, a fluorescence image and a transmitted light image of each nucleated cell. One nucleated cell was captured in each acquired fluorescence image and each transmitted light image. First, based on the optical signal of each cell, a 2D scattergram with the fluorescence intensity (CD33 label) of PerCP/Cy5.5 (trademark) on the X-axis and the side scattered light intensity on the Y-axis was created. Fig. 8 shows an example of the created 2D scattergram. In the created 2D scattergram, a cell having a fluorescence intensity and a side scattered light intensity each within the predetermined range was selected and extracted as a monocyte fraction. In Fig. 8, each cell in the area surrounded by an ellipse represents a monocyte.

### (2.2) Monocyte image analyzing

The fluorescence image and the transmitted light image of each monocyte in the monocyte fraction extracted in the above-mentioned (2.1) were confirmed. An example of the image is shown in Fig. 9. In the drawing, "BF" is a bright-field image (transmitted light image), and "Merge" is a superposition of a fluorescence image of coilin and a fluorescence image of an SMN protein. From Fig. 9, it was found that each of coilin and an SMN protein was imaged as one bright spot. In Fig. 9, an SMN protein present in one monocyte has one bright spot, but there is a cell having two or more bright spots. With reference to Merge in Fig. 9, there were a cell in which the intracellular distance between the bright spot of an SMN protein and the bright spot of coilin is large like the cell in (a), and a cell in which the intracellular distance between the bright spot of coilin and the bright spot of an SMN protein is close like the cell in (b).

Based on the four indicators shown in Table 1, the healthy subject and the SMA patient were compared for a monocyte in the measurement sample. The ratio for each indicator is a ratio of the number of predetermined monocytes shown in Table 1 to the number of monocytes in the extracted fraction. The position where the bright spot of an SMN protein and the bright spot of coilin are close to each other was determined as follows. In the fluorescence image, the distance between the centers of gravity of the bright spot of an SMN protein and the bright spot of coilin in one monocyte is calculated, and when the distance is 0.8 µm or less, it is determined that both bright spots are close to each other.

**[Table 1]**

| Fig. 10 | Indicator |
|---|---|
| A | Ratio of monocyte having one or more bright spots of SMN protein |
| B | Ratio of monocyte having two or more bright spots of SMN protein |
| C | Ratio of monocyte having three or more bright spots of SMN protein |
| D | Ratio of monocyte having one or more positions where bright spot of SMN protein and bright spot of coilin are close to each other |

### (3) Result

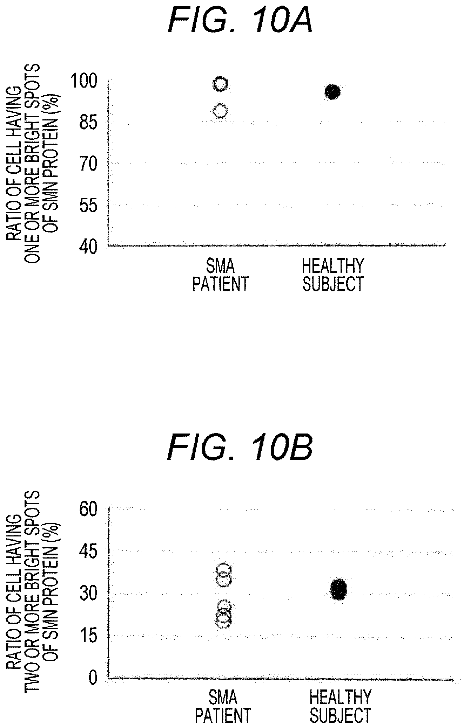

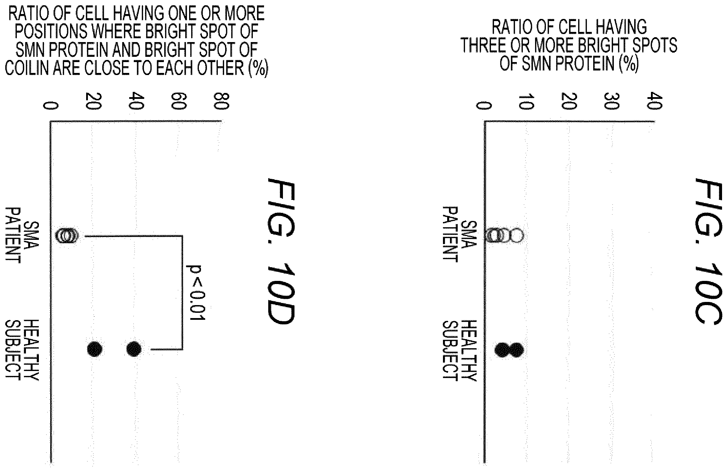

The comparison results based on each of the indicators are shown in Figs. 10A to 10D. As can be seen from Figs. 10A to 10C, there was no significant difference in the number of bright spots of SMN protein in a monocyte between the healthy subjects and the SMA patients. On the other hand, as shown in Fig. 10D, there is a difference between healthy subjects and the SMA patients in the ratio of a monocyte having one or more positions where the bright spot of an SMN protein and the bright spot of coilin are close to each other. For this ratio, the mean value and standard deviation (SD) of the SMA patients were calculated. When determining whether or not a subject is an SMA patient using a value of the mean value plus 3SD (12.1%) as the cutoff value, it was possible to distinguish between the healthy subjects and the SMA patients at a p-value of less than 0.01. These results suggest that the intracellular distance between an SMN protein and coilin localized in the nucleus of a monocyte can be used as an indicator to acquire information whether or not a subject suffers from SMA.

### Example 2: Determining response of treatment for SMA

### (1) Preparation of measurement sample

To one SMA patient, Spinraza (trademark) (generic name: Nusinersen, Biogen) was administered 4 times. The administration schedule was performed 4 times in total according to the package insert. From this patient, peripheral blood was collected into a heparin-containing blood collection tube before administration, the day before the first administration, and the day before the fourth administration. From the collected peripheral blood, a measurement sample was prepared in the same manner as in Example 1. From peripheral blood collected before administration, immediately before the second administration, immediately before the third administration, and immediately before the fourth administration, sera were obtained.

### (2) Cell measuring and image analyzing

In the same manner as in Example 1, each measurement sample was measured and imaged with MI-1000 (Sysmex Corporation), and the image was analyzed. For each measurement sample, the ratio of a monocyte having one or more positions where the bright spot of an SMN protein and the bright spot of coilin to the number of monocytes in an extracted fraction were close to each other was calculated.

### (3) Measuring of serum creatinine kinase activity

Creatine kinase (CK) activity in the serum was measured. The measurement was performed using Labospect008 or Labospect008α (Hitachi High-Tech Corporation) and Cygnus Auto CK (Shino-Test Corporation) according to the package insert. It is known that CK activity is a biomarker indicating the degree of progression of SMA, and CK activity decreases as SMA progresses.

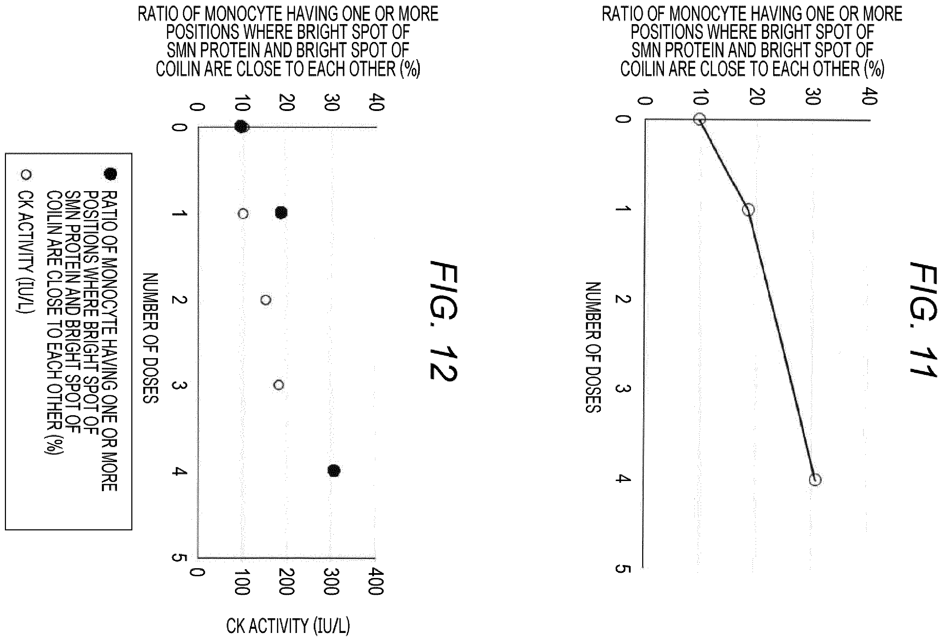

The result of image analysis is shown in Fig. 11. In the drawing, the value (12.1%) calculated in Example 1 is shown as the cutoff value. In Fig. 12, the result of serum CK activity measurement is shown on the graph of Fig. 11. As can be seen from Fig. 11, the first dose of Spinraza (trademark) showed a value that exceeded the cutoff value. After the 4th dose, the value increased further. Therefore, it was indicated that the treatment with Spinraza (trademark) was highly likely to be successful in the patient. As shown in Fig. 12, serum CK activity increased after the second administration of Spinraza (trademark). Since CK activity increases when muscle is moved, it can be seen that the administration of Spinraza (trademark) makes the patient more able to move muscle, so that the motor function is highly likely to be improved. This result suggests that the intracellular distance between an SMN protein and coilin localized in the nucleus of a monocyte can be used as an indicator to acquire information on the response of a treatment for SMA.

## Claims

1. A method for acquiring information on spinal muscular atrophy, comprising
acquiring a fluorescence image of a monocyte in a measurement sample, wherein the measurement sample is a sample prepared from a blood specimen that has been obtained from a subject whereby preparing the sample comprises labeling an SMN protein in the monocyte with a first fluorescent dye, and labeling coilin in the monocyte with a second fluorescent dye,
determining an intracellular distance between a first bright spot corresponding to the first fluorescent dye and a second bright spot corresponding to the second fluorescent dye in the fluorescence image, and
acquiring a value regarding a number of monocytes in which the intracellular distance is equal to or less than a first threshold value,
wherein the value is an indicator of spinal muscular atrophy affection, wherein:
when the value is less than a second threshold value, it is suggested that the subject suffers from spinal muscular atrophy; or
when the value is equal to or higher than a second threshold value, it is suggested that the subject does not suffer from spinal muscular atrophy.

2. A method for acquiring information on spinal muscular atrophy, comprising
acquiring a fluorescence image of a monocyte in a measurement sample, wherein the measurement sample is a sample prepared from a blood specimen that has been obtained from a patient who has undergone a treatment for spinal muscular atrophy, whereby preparing the sample comprises labeling an SMN protein in the monocyte with a first fluorescent dye, and labeling coilin in the monocyte with a second fluorescent dye,
determining an intracellular distance between a first bright spot corresponding to the first fluorescent dye and a second bright spot corresponding to the second fluorescent dye in the fluorescence image, and
acquiring a value regarding a number of monocytes in which the intracellular distance is equal to or less than a first threshold value,
wherein the value is an indicator of a response of the treatment for spinal muscular atrophy, wherein:
when the value is less than a second threshold value, it is suggested that the treatment for spinal muscular atrophy is not successful in the patient; or
when the value is equal to or higher than a second threshold value, it is suggested that the treatment for spinal muscular atrophy is successful in the patient.

3. A method for acquiring information on spinal muscular atrophy, comprising
acquiring a first fluorescence image of a monocyte in a first measurement sample and a second fluorescence image of a monocyte in a second measurement sample, wherein the first measurement sample is prepared from a blood specimen that has been obtained from a patient before receiving a treatment for spinal muscular atrophy and a second measurement sample is prepared from a blood specimen that has been obtained from the patient after receiving the treatment, whereby preparing the first and second measurement samples comprises labeling an SMN protein in the monocyte with a first fluorescent dye, and labeling coilin in the monocyte with a second fluorescent dye,
determining a first intracellular distance between a first bright spot corresponding to the first fluorescent dye and a second bright spot corresponding to the second fluorescent dye in the first fluorescence image, and determining second intracellular distance between a first bright spot corresponding to the first fluorescent dye and a second bright spot corresponding to the second fluorescent dye in the second fluorescence image, and
acquiring a first value regarding a number of monocytes in which the first intracellular distance is equal to or less than a first threshold value, and a second value regarding a number of monocytes in which the second intracellular distance is equal to or less than a second threshold value,
wherein the first and second values are indicators of a response of the treatment for spinal muscular atrophy, wherein:
when the second value is less than the first value, it is suggested that the treatment for spinal muscular atrophy is not successful in the patient, or
when the second value is equal to or greater than the first value, it is suggested that the treatment for spinal muscular atrophy is successful in the patient.

4. The method according to any one of claims 1 to 3, wherein the intracellular distance is a distance between a center of gravity of the first bright spot and a center of gravity of the second bright spot in the fluorescence image.

5. The method according to any one of claims 1 to 4, wherein in the determining of an intracellular distance, when a plurality of the first bright spots and/or the second bright spots are present in one monocyte, a distance between the closest first bright spot and second bright spot is determined as the intracellular distance.

6. The method according to any one of claims 1 to 5, wherein in the determining of an intracellular distance, when a plurality of the first bright spots and/or the second bright spots are present in one monocyte, distances between the first bright spot and second bright spot for all combinations of the first bright spot and second bright spot are determined, and the distances are compared to determine a shortest distance among the distances as the intracellular distance.

7. The method according to any one of claims 1 to 6, further comprising acquiring a number of monocytes comprising at least one first bright spot and at least one second bright spot in the fluorescence image,
wherein the value is a ratio or a value of the ratio of a number of monocytes in which the intracellular distance is equal to or less than a first threshold value to the acquired number of monocytes.

## Patentansprüche

1. Verfahren zur Erfassung von Informationen über spinale Muskelatrophie, umfassend
Erfassen eines Fluoreszenzbildes eines Monozyten in einer Messprobe, wobei die Messprobe eine Probe ist, die aus einer Blutmuster hergestellt wurde, die einem Subjekt entnommen wurde, wobei das Herstellen der Probe Markieren eines SMN-Proteins in dem Monozyten mit einem ersten Fluoreszenzfarbstoff und Markieren von Coilin in dem Monozyten mit einem zweiten Fluoreszenzfarbstoff umfasst,
Bestimmen eines intrazellulären Abstands zwischen einem ersten hellen Fleck, der dem ersten Fluoreszenzfarbstoff entspricht, und einem zweiten hellen Fleck, der dem zweiten Fluoreszenzfarbstoff entspricht, in dem Fluoreszenzbild, und
Erfassen eines Wertes bezüglich einer Anzahl von Monozyten, in denen der intrazelluläre Abstand gleich einem ersten Schwellenwert oder geringer als dieser ist,
wobei der Wert ein Hinweis auf Erkrankung an spinaler Muskelatrophie ist, wobei:
wenn der Wert geringer ist als ein zweiter Schwellenwert, dies darauf hinweist, dass das Subjekt an spinaler Muskelatrophie leidet, oder,
wenn der Wert gleich einem zweiten Schwellenwert oder höher als dieser ist, dies darauf hinweist, dass das Subjekt nicht an spinaler Muskelatrophie leidet.

2. Verfahren zur Erfassung von Informationen über spinale Muskelatrophie, umfassend
Erfassen eines Fluoreszenzbildes eines Monozyten in einer Messprobe, wobei die Messprobe eine Probe ist, die aus einer Blutmuster hergestellt wurde, die einem Patienten entnommen wurde, der sich einer Behandlung für spinale Muskelatrophie unterzogen hat, wobei Herstellen der Probe Markieren eines SMN-Proteins in dem Monozyten mit einem ersten Fluoreszenzfarbstoff und Markieren von Coilin in dem Monozyten mit einem zweiten Fluoreszenzfarbstoff umfasst,
Bestimmen eines intrazellulären Abstands zwischen einem ersten hellen Fleck, der dem ersten Fluoreszenzfarbstoff entspricht, und einem zweiten hellen Fleck, der dem zweiten Fluoreszenzfarbstoff entspricht, in dem Fluoreszenzbild, und
Erfassen eines Wertes bezüglich einer Anzahl von Monozyten, in denen der intrazelluläre Abstand gleich einem ersten Schwellenwert oder geringer als dieser ist,
wobei der Wert ein Hinweis auf ein Ansprechen auf die Behandlung für spinale Muskelatrophie ist, wobei:
wenn der Wert geringer ist als ein zweiter Schwellenwert, dies darauf hinweist, dass die Behandlung für spinale Muskelatrophie bei dem Patienten nicht erfolgreich ist, oder,
wenn der Wert gleich einem zweiten Schwellenwert oder höher als dieser ist, dies darauf hinweist, dass die Behandlung für spinale Muskelatrophie bei dem Patienten erfolgreich ist.

3. Verfahren zur Erfassung von Informationen über spinale Muskelatrophie, umfassend
Erfassen eines ersten Fluoreszenzbildes eines Monozyten in einer ersten Messprobe und eines zweiten Fluoreszenzbildes eines Monozyten in einer zweiten Messprobe, wobei die erste Messprobe aus einer Blutmuster hergestellt wurde, die einem Patienten entnommen wurde, bevor er eine Behandlung für spinale Muskelatrophie erhielt, und eine zweite Messprobe aus einer Blutmuster hergestellt wurde, die dem Patienten entnommen wurde, nachdem er die Behandlung erhalten hat, wobei Herstellen der ersten und zweiten Messprobe Markieren von SMN-Protein in dem Monozyten mit einem ersten Fluoreszenzfarbstoff und Markieren von Coilin in dem Monozyten mit einem zweiten Fluoreszenzfarbstoff umfasst,
Bestimmen eines ersten intrazellulären Abstands zwischen einem ersten hellen Fleck, der dem ersten Fluoreszenzfarbstoff entspricht, und einem zweiten hellen Fleck, der dem zweiten Fluoreszenzfarbstoff entspricht, in dem ersten Fluoreszenzbild, und Bestimmen eines zweiten intrazellulären Abstands zwischen einem ersten hellen Fleck, der dem ersten Fluoreszenzfarbstoff entspricht, und einem zweiten hellen Fleck, der dem zweiten Fluoreszenzfarbstoff entspricht, in dem zweiten Fluoreszenzbild, und
Erfassen eines ersten Wertes bezüglich einer Anzahl von Monozyten, in denen der erste intrazelluläre Abstand gleich einem ersten Schwellenwert oder geringer als dieser ist, und eines zweiten Wertes bezüglich einer Anzahl von Monozyten, in denen der zweite intrazelluläre Abstand gleich einem zweiten Schwellenwert oder geringer als dieser ist,
wobei die ersten und zweiten Werte Hinweise auf ein Ansprechen auf die Behandlung für spinale Muskelatrophie sind, wobei:
wenn der zweite Wert geringer ist als der erste Wert, dies darauf hinweist, dass die Behandlung für spinale Muskelatrophie bei dem Patienten nicht erfolgreich ist, oder,
wenn der zweite Wert gleich dem erste Wert oder größer als dieser ist, dies darauf hinweist, dass die Behandlung für spinale Muskelatrophie bei dem Patienten erfolgreich ist.

4. Verfahren nach einem der Ansprüche 1 bis 3, wobei der intrazelluläre Abstand ein Abstand zwischen einem Schwerpunkt des ersten hellen Flecks und einem Schwerpunkt des zweiten hellen Flecks in dem Fluoreszenzbild ist.

5. Verfahren nach einem der Ansprüche 1 bis 4, wobei beim Bestimmen eines intrazellulären Abstands, wenn eine Vielzahl der ersten hellen Flecken und/oder der zweiten hellen Flecken in einem Monozyten vorhanden sind, ein Abstand zwischen dem ersten hellen Fleck und dem zweiten hellen Fleck, die am nächsten beieinander liegen, als der intrazelluläre Abstand erfasst wird.

6. Verfahren nach einem der Ansprüche 1 bis 5, wobei beim Bestimmen eines intrazellulären Abstands, wenn eine Vielzahl der ersten hellen Flecken und/oder der zweiten hellen Flecken in einem Monozyten vorhanden sind, Abstände zwischen dem ersten hellen Fleck und zweiten hellen Fleck für alle Kombinationen des ersten hellen Flecks und zweiten hellen Flecks bestimmt werden,
und die Abstände verglichen werden, um einen kürzesten Abstand unter den Abständen als den intrazellulären Abstand zu erfassen.

7. Verfahren nach einem der Ansprüche 1 bis 6, ferner umfassend Erfassen einer Anzahl von Monozyten, die wenigstens einen ersten hellen Fleck und wenigstens einen zweiten hellen Fleck in dem Fluoreszenzbild umfassen,
wobei der Wert ein Verhältnis oder ein Wert des Verhältnisses einer Anzahl von Monozyten, in denen der intrazelluläre Abstand gleich einem ersten Schwellenwert oder geringer als dieser ist, zu der erfassten Anzahl von Monozyten ist.

## Revendications

1. Procédé pour l'acquisition d'informations sur l'atrophie musculaire spinale, comprenant
l'acquisition d'une image de fluorescence d'un monocyte dans un échantillon de mesure, l'échantillon de mesure étant un échantillon préparé à partir d'un spécimen de sang qui a été obtenu d'un sujet, la préparation de l'échantillon comprenant le marquage d'une protéine SMN dans le monocyte par un premier colorant fluorescent, et le marquage de la colline dans le monocyte par un deuxième colorant fluorescent,
la détermination d'une distance intracellulaire entre un premier point lumineux correspondant au premier colorant fluorescent et un deuxième point lumineux correspondant au deuxième colorant fluorescent dans l'image de fluorescence, et
l'acquisition d'une valeur concernant un nombre de monocytes dans lesquels la distance intracellulaire est égale ou inférieure à une première valeur seuil,
la valeur étant un indicateur d'une affection de type atrophie musculaire spinale, dans lequel :
lorsque la valeur est inférieure à une deuxième valeur seuil, il est suggéré que le sujet souffre d'atrophie musculaire spinale ; ou
lorsque la valeur est égale ou supérieure à une deuxième valeur seuil, il est suggéré que le sujet ne souffre pas d'atrophie musculaire spinale.

2. Procédé pour l'acquisition d'informations sur l'atrophie musculaire spinale, comprenant
l'acquisition d'une image de fluorescence d'un monocyte dans un échantillon de mesure, l'échantillon de mesure étant un échantillon préparé à partir d'un spécimen de sang qui a été obtenu d'un patient qui a subi un traitement pour une atrophie musculaire spinale, la préparation de l'échantillon comprenant le marquage d'une protéine SMN dans le monocyte par un premier colorant fluorescent, et le marquage de la colline dans le monocyte par un deuxième colorant fluorescent,
la détermination d'une distance intracellulaire entre un premier point lumineux correspondant au premier colorant fluorescent et un deuxième point lumineux correspondant au deuxième colorant fluorescent dans l'image de fluorescence, et
l'acquisition d'une valeur concernant un nombre de monocytes dans lesquels la distance intracellulaire est égale ou inférieure à une première valeur seuil,
la valeur étant un indicateur d'une réponse du traitement pour une atrophie musculaire spinale, dans lequel :
lorsque la valeur est inférieure à une deuxième valeur seuil, il est suggéré que le traitement pour une atrophie musculaire spinale ne réussit pas chez le patient ; ou
lorsque la valeur est égale ou supérieure à une deuxième valeur seuil, il est suggéré que le traitement pour une atrophie musculaire spinale réussit chez le patient.

3. Procédé pour l'acquisition d'informations sur une atrophie musculaire spinale, comprenant
l'acquisition d'une première image de fluorescence d'un monocyte dans un premier échantillon de mesure et d'une deuxième image de fluorescence d'un monocyte dans un deuxième échantillon de mesure, le premier échantillon de mesure étant préparé à partir d'un spécimen de sang qui a été obtenu d'un patient avant de recevoir un traitement pour une atrophie musculaire spinale et un deuxième échantillon de mesure étant préparé à partir d'un spécimen de sang qui a été obtenu du patient après réception du traitement, la préparation des premier et deuxième échantillons de mesure comprenant le marquage d'une protéine SMN dans le monocyte par un premier colorant fluorescent, et le marquage de la colline dans le monocyte par un deuxième colorant fluorescent,
la détermination d'une première distance intracellulaire entre un premier point lumineux correspondant au premier colorant fluorescent et un deuxième point lumineux correspondant au deuxième colorant fluorescent dans la première image de fluorescence, et la détermination d'une deuxième distance intracellulaire entre un premier point lumineux correspondant au premier colorant fluorescent et un deuxième point lumineux correspondant au deuxième colorant fluorescent dans la deuxième image de fluorescence, et
l'acquisition d'une première valeur concernant un nombre de monocytes dans lesquels la première distance intracellulaire est égale ou inférieure à une première valeur seuil, et d'une deuxième valeur concernant un nombre de monocytes dans lesquels la deuxième distance intracellulaire est égale ou inférieure à une deuxième valeur seuil,
la première et la deuxième valeur étant des indicateurs d'une réponse du traitement pour une atrophie musculaire spinale, dans lequel :
lorsque la deuxième valeur est inférieure à la première valeur, il est suggéré que le traitement pour une atrophie musculaire spinale ne réussit pas chez le patient, ou
lorsque la deuxième valeur est égale ou supérieure à la première valeur, il est suggéré que le traitement pour une atrophie musculaire spinale réussit chez le patient.

4. Procédé selon l'une quelconque des revendications 1 à 3, la distance intracellulaire étant une distance entre un centre de gravité du premier point lumineux et un centre de gravité du deuxième point lumineux dans l'image de fluorescence.

5. Procédé selon l'une quelconque des revendications 1 à 4, dans la détermination d'une distance intracellulaire, lorsqu'une pluralité des premiers points lumineux et/ou des deuxièmes points lumineux sont présents dans un monocyte, une distance entre le premier point lumineux et le deuxième point lumineux, qui sont les plus proches l'un de l'autre, étant acquise en tant que la distance intracellulaire.

6. Procédé selon l'une quelconque des revendications 1 à 5, dans la détermination d'une distance intracellulaire, lorsqu'une pluralité des premiers points lumineux et/ou des deuxièmes points lumineux sont présents dans un monocyte, des distances entre le premier point lumineux et le deuxième point lumineux pour toutes les combinaisons du premier point lumineux et du deuxième point lumineux étant déterminées, et les distances étant comparées pour acquérir une distance la plus courte parmi les distances en tant que la distance intracellulaire.

7. Procédé selon l'une quelconque des revendications 1 à 6, comprenant en outre l'acquisition d'un nombre de monocytes comprenant au moins un premier point lumineux et au moins un deuxième point lumineux dans l'image de fluorescence,
la valeur étant un rapport ou une valeur du rapport d'un nombre de monocytes dans lesquels la distance intracellulaire est égale ou inférieure à une première valeur seuil sur le nombre acquis de monocytes.
